# EUROPEAN PATENT SPECIFICATION

(11) **EP 4 472 087 B1**
(45) Date of publication and mention of the grant of the patent: **06.05.2026**
(21) Application number: 23773789.5
(22) Date of filing: 20.03.2023
(51) Int. Cl.: H04B 1/3827, A61B 5/01, A61B 5/1172, A61B 5/11, A61B 5/024, H04M 1/72409, A61B 5/00, G01K 13/20, G01D 21/02

(54) **FEVER ALARM METHOD BASED ON BODY TEMPERATURE MONITORING, AND RELATED DEVICE**
FIEBERALARMVERFAHREN AUF BASIS VON KÖRPERTEMPERATURÜBERWACHUNG UND ZUGEHÖRIGE VORRICHTUNG
PROCÉDÉ D'ALARME DE FIÈVRE BASÉ SUR LA SURVEILLANCE DE LA TEMPÉRATURE CORPORELLE, ET DISPOSITIF ASSOCIÉ

(30) Priority: 23.03.2022 CN 202210290825
(43) Date of publication of application: 04.12.2024
(73) Proprietor: Huawei Technologies Co., Ltd., Shenzhen, Guangdong 518129 (CN)
(72) Inventor: WANG, Lu, Shenzhen, Guangdong 518129 (CN); SHENG, Rong, Shenzhen, Guangdong 518129 (CN); HAN, Yujia, Shenzhen, Guangdong 518129 (CN); WANG, Runsen, Shenzhen, Guangdong 518129 (CN); HE, Zhijian, Shenzhen, Guangdong 518129 (CN)
(74) Representative: Körber, Martin Hans
(86) International application number: PCT/CN2023/082515
(87) International publication number: WO 2023/179541

(56) References cited:
- WO-A1-2021/213337
- CN-A- 109 124 603
- CN-A- 111 412 992
- CN-A- 112 577 611
- CN-A- 113 170 957
- CN-A- 113 827 185
- US-A1- 2013 072 765
- US-A1- 2021 278 290
- US-A1- 2021 353 236

## Description

### TECHNICAL FIELD

This application relates to the field of computer technologies, and in particular, to a fever alarm method based on body temperature monitoring and a related device.

### BACKGROUND

A body temperature is a very important physiological sign of human body and a necessary condition for ensuring normal metabolism and life activities. Occurrences of some diseases are often accompanied by an abnormal body temperature. In addition to in initial screening of diseases, the body temperature also plays an important role in many application scenarios such as health and fitness, living habits, and smart home.

At present, temperature measurement products include a digital thermometer, an ear thermometer, a forehead thermometer, a body temperature sticker, a temperature measurement bracelet, and the like, and temperature measurement principles of the temperature measurement products are all based on one or more of a thermistor, infrared heat radiation, and a heat flux. However, devices such as the digital thermometer, the ear thermometer, and the forehead thermometer are all used to measure a user's body temperature once, and are not suitable for a scenario in which long-term continuous body temperature monitoring is required. Most of body temperature stickers and temperature measurement bracelets use the temperature measurement principle of thermistor. This requires algorithm compensation, and cannot guarantee accuracy of body temperature detection.

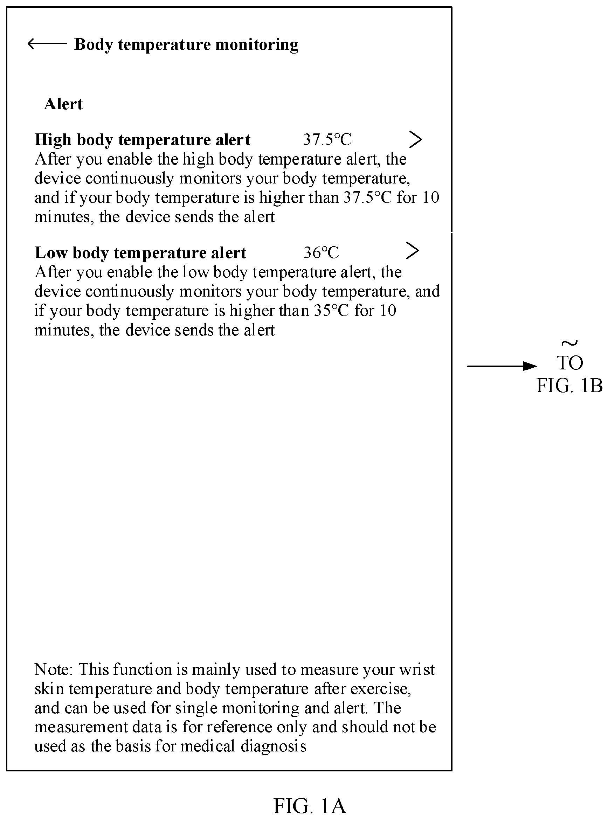

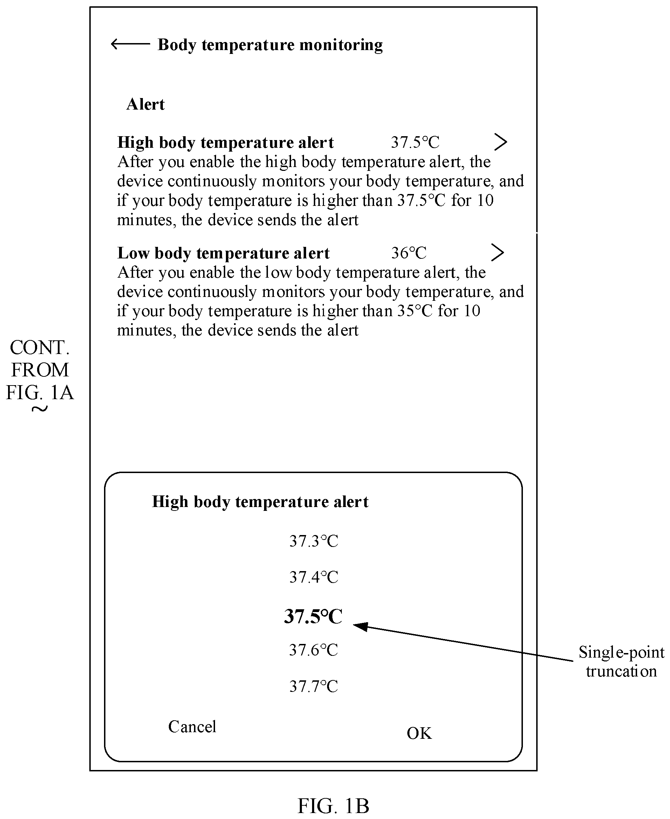

Usually, FIG. 1A and FIG. 1B are a diagram of a single-point truncated alarm according to an embodiment of this application. It can be seen from FIG. 1A and FIG. 1B that a wearable device like a body temperature sticker or a temperature measurement bracelet usually uses a single-point truncated alarm mechanism, that is, a body temperature alarm threshold (for example, 37.5°C) is set inside an application (application, APP). When a body temperature measured by the wearable device like the body temperature sticker or the temperature measurement bracelet is higher than the threshold, a fever alarm is generated. However, due to an environmental factor and an individual difference, a body temperature of a user may be higher than 37.5°C in some scenarios. As a result, there is a high probability that the single-point truncated alarm mechanism generates a false fever alarm, causing a puzzle and a trouble to the user, leading to an incorrect misunderstanding of the body temperature by the user, and failing to meet requirements of different users.

US 2013/0072765 A1 discloses a body-worn device comprising an accelerometer and a temperature sensor. Further, it includes a power management system to move the body-worn device into a low power state when the body-worn device is not being worn, the power management system determining when the body-worn device is worn based on a combination of sensor data.

US 2021/0353236 A1 discloses a system and method for a wearable device for alerting of body temperature of the wearer.

US 2021/0278290 A1 discloses a temperature sensor and fever alert generator with tunable parameters.

### SUMMARY OF THE INVENTION

Embodiments of this application provide a fever alarm method based on body temperature monitoring and a related device, so that when a temperature monitoring mode that a user is in is determined, a fever alarm can be generated after it is detected that a body temperature of the user meets a fever condition corresponding to the body temperature monitoring mode. This improves accuracy of the fever alarm. The invention is defined in the independent claims. Additional features of the invention are provided in the dependent claims. In the following, parts of the description and drawings referring to embodiments which are not covered by the claims are not presented as embodiments of the invention, but as examples useful for understanding the invention.

### SUMMARY OF THE DISCLOSURE

According to a first aspect, an embodiment of this application provides a fever alarm method based on body temperature monitoring, applied to a wearable device. The method may include: collecting a body temperature of a user in a first time period, where the first time period is time for which the wearable device is attached to skin of the user; determining a body temperature monitoring mode that the user is in; and generating a fever alarm when detecting that the body temperature of the user in the first time period meets a fever condition corresponding to the body temperature monitoring mode, where the fever condition includes one or more body temperatures and time corresponding to each body temperature, and different body temperature monitoring modes correspond to different fever conditions.

In this application, the wearable device may collect the body temperature of the user. After determining the body temperature monitoring mode that the user is in, the wearable device monitors whether the body temperature of the user meets the fever condition corresponding to the body temperature monitoring mode. If it is detected that the body temperature of the user meets the fever condition corresponding to the body temperature monitoring mode, it indicates that the user has a fever, and the fever alarm is generated. Different from an existing single-point truncated determining manner, the different body temperature monitoring modes in this application correspond to the different fever conditions. In this way, a case in which body temperatures of some users may be higher than a specific fever temperature in some scenarios due to an environmental factor and an individual difference can be avoided. Therefore, a risk of a false fever alarm can be reduced, and user experience can be improved.

In a possible implementation, the body temperature detection mode includes one or more of the following: an intelligent mode, a daily mode, a sleep mode, and an exercise mode.

In this application, the fever alarm based on the body temperature monitoring may cover scenarios such as daily home, office, sleep, and exercise, and different scenarios correspond to different fever conditions. A more accurate fever alarm can be provided through division into scenarios. A time dimension is introduced based on a segment threshold. Each body temperature segment corresponds to different duration, and the fever alarm is generated only when a duration limit is met, thereby reducing a risk of a false alarm, and improving user experience.

In a possible implementation, if the body temperature monitoring mode that the user is in is the intelligent mode, the generating a fever alarm when detecting that the body temperature of the user in the first time period meets a fever condition corresponding to the body temperature monitoring mode includes: in a case of detecting that time for which the body temperature of the user in the first time period exceeds a first body temperature is greater than first time, when detecting that time for which the body temperature of the user in the first time period exceeds a third body temperature is greater than third time, generating the fever alarm, where the third body temperature is greater than the first body temperature, and the third time is greater than or equal to the first time.

In a possible implementation, the method further includes: in a case of detecting that the time for which the body temperature of the user in the first time period exceeds the first body temperature is greater than the first time, when detecting that time for which the body temperature of the user in the first time period exceeds a second body temperature is greater than second time, generating the fever alarm, where the third body temperature is greater than the second body temperature, and the second time is greater than the first time.

In a possible implementation, the method further includes: in a case of detecting that the time for which the body temperature of the user in the first time period exceeds the first body temperature is greater than the first time, when detecting that time for which the body temperature of the user in the first time period exceeds a second body temperature is less than second time, determining that a current state of the user is a sleep state; and in the sleep state, generating the fever alarm when detecting that the time for which the body temperature of the user in the first time period exceeds the first body temperature is greater than the fourth time, where the fourth time is greater than the first time.

In a possible implementation, if the body temperature monitoring mode that the user is in is the intelligent mode, the generating a fever alarm when detecting that the body temperature of the user in the first time period meets a fever condition corresponding to the body temperature monitoring mode includes: in a case of detecting that duration of the first body temperature of the user in the first time period is greater than the first time, when detecting that the time for which the body temperature of the user in the first time period exceeds the second body temperature is less than the second time, determining that the current state of the user is an exercise state; and in the exercise state, generating the fever alarm when detecting that the first body temperature of the user in the first time period is greater than fifth time, where the fifth time is greater than the first time.

In a possible implementation, if the body temperature monitoring mode that the user is in is the intelligent mode, the generating a fever alarm when detecting that the body temperature of the user in the first time period meets a fever condition corresponding to the body temperature monitoring mode includes: in a case of detecting that the duration of the first body temperature of the user in the first time period is greater than the first time, when detecting that the time for which the body temperature of the user in the first time period exceeds the second body temperature is less than the second time, determining that the current state of the user is a daily state; and in the daily state, generating the fever alarm when detecting that the first body temperature of the user in the first time period is greater than sixth time, where the sixth time is greater than the first time.

It can be seen that in the intelligent mode, a time dimension is introduced based on a segment threshold. Each body temperature segment corresponds to different duration, and the fever alarm is generated only when a duration limit is met. In addition, impact of disturbing factors such as the sleep state and the exercise state is further considered, and the sleep state and the exercise state are determined. The fever alarm is generated when the fever condition corresponding to the sleep state in the intelligent mode is met, or when the fever condition corresponding to the sleep state in the intelligent mode is met. Therefore, a risk of a false alarm can be reduced, and user experience can be improved.

In a possible implementation, if the body temperature monitoring mode that the user is in is the sleep mode, the generating a fever alarm when detecting that the body temperature of the user in the first time period meets a fever condition corresponding to the body temperature monitoring mode includes: determining, based on a setting of the user, one or more first abnormal body temperatures corresponding to the sleep mode and first duration corresponding to the first abnormal body temperature; adjusting the first abnormal body temperature or the first duration based on a detected ambient temperature, to obtain a second abnormal body temperature or second duration; and generating the fever alarm when detecting that time for which the body temperature of the user in the first time period exceeds the first abnormal body temperature is greater than the second duration; or generating the fever alarm when detecting that time for which the body temperature of the user in the first time period exceeds the second abnormal body temperature is greater than the first duration.

In a possible implementation, the adjusting the first abnormal body temperature or the first duration based on a detected ambient temperature, to obtain a second abnormal body temperature or second duration includes: decreasing the first abnormal body temperature to obtain the second abnormal body temperature, or prolonging the first duration to obtain the second duration when detecting that the ambient temperature decreases in preset time; and increasing the first abnormal body temperature to obtain the second abnormal body temperature, or shortening the first duration to obtain the second duration when detecting that the ambient temperature increases in the preset time.

It can be seen that, in the sleep mode, the abnormal body temperature and the duration that correspond to the sleep mode can be adaptively adjusted based on the ambient temperature, thereby reducing impact of an environmental factor on the fever alarm, reducing a risk of a false alarm, and improving user experience.

In a possible implementation, if the body temperature monitoring mode that the user is in is the exercise mode, the generating a fever alarm when detecting that the body temperature of the user in the first time period meets a fever condition corresponding to the body temperature monitoring mode includes:
determining, based on a setting of the user, one or more third abnormal body temperatures corresponding to the exercise mode and third duration corresponding to the third abnormal body temperature; adjusting the third abnormal body temperature or the third duration based on a detected exercise intensity of the user, to obtain a fourth abnormal body temperature or fourth duration, where the third abnormal body temperature is less than or equal to the fourth abnormal body temperature, and the third duration is less than the fourth duration; and generating the fever alarm when detecting that time for which the body temperature of the user in the first time period exceeds the third abnormal body temperature is greater than the fourth duration; or generating the fever alarm when detecting that time for which the body temperature of the user in the first time period exceeds the fourth abnormal body temperature is greater than the third duration.

It can be seen that, in the exercise mode, the abnormal body temperature and the duration that correspond to the exercise mode can be adaptively adjusted based on an exercise state of the user, thereby reducing a risk of a false alarm caused by a body temperature increase brought by exercise, and improving user experience.

According to a second aspect, an embodiment of this application provides a wearable device, including a transceiver, a processor, and a memory. The memory is configured to store a computer program, and the processor invokes the computer program to perform the fever alarm method based on body temperature monitoring according to the first aspect and any one of the implementations of the first aspect of embodiments of this application.

According to a third aspect, an embodiment of this application provides a computer storage medium. The computer storage medium stores a computer program, and when the computer program is executed by a processor, the fever alarm method based on body temperature monitoring according to the first aspect and any one of the implementations of the first aspect of embodiments of this application is implemented.

According to a fourth aspect, an embodiment of this application provides a computer program product. When the computer program product runs on an electronic device, the electronic device is enabled to perform the fever alarm method based on body temperature monitoring according to the first aspect and any one of the implementations of the first aspect of embodiments of this application.

It should be understood that descriptions of technical features, technical solutions, beneficial effect, or similar languages in this application do not imply that all features and advantages can be implemented in any single embodiment. On the contrary, it may be understood that the descriptions of features or beneficial effect indicate that at least one embodiment includes a specific technical feature, technical solution, or beneficial effect. Therefore, the descriptions of technical features, technical solutions, or beneficial effect in this specification do not necessarily refer to a same embodiment. Further, the technical features, technical solutions, and beneficial effect described in embodiments may be combined in any proper manner. A person skilled in the art may understand that an embodiment may be implemented without one or more specific technical features, technical solutions, or beneficial effect in a specific embodiment. In other embodiments, additional technical features and beneficial effect may also be identified in a specific embodiment that does not reflect all embodiments.

### BRIEF DESCRIPTION OF DRAWINGS

The following describes accompanying drawings used in embodiments of this application.
FIG. 1A and FIG. 1B are a diagram of a single-point truncated alarm according to an embodiment of this application;
FIG. 2A and FIG. 2B are a diagram of a scenario of a fever alarm based on body temperature detection according to an embodiment of this application;
FIG. 3A is a schematic functional block diagram of a wearable device according to an embodiment of this application;
FIG. 3B is a diagram of a software architecture of a wearable device according to an embodiment of this application;
FIG. 3C is a diagram of a structure of a wearable device according to an embodiment of this application;
FIG. 4A is a diagram of enabling a body temperature monitoring mode by a wearable device according to an embodiment of this application;
FIG. 4B-1 to FIG. 4B-4 are another diagram of enabling a body temperature monitoring mode by a wearable device according to an embodiment of this application;

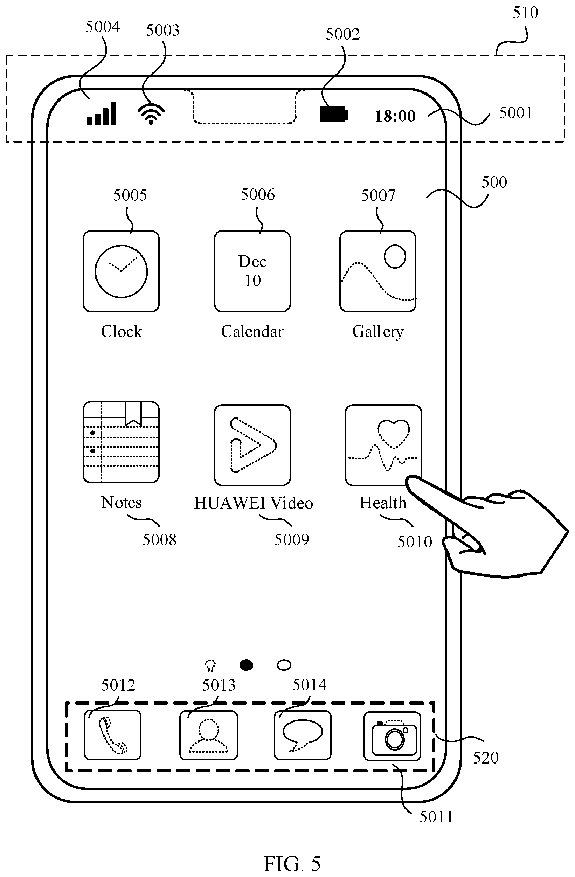

FIG. 5 is a diagram of receiving a request for enabling a body temperature monitoring mode sent by a terminal device 200 to enable the body temperature monitoring mode according to an embodiment of this application;

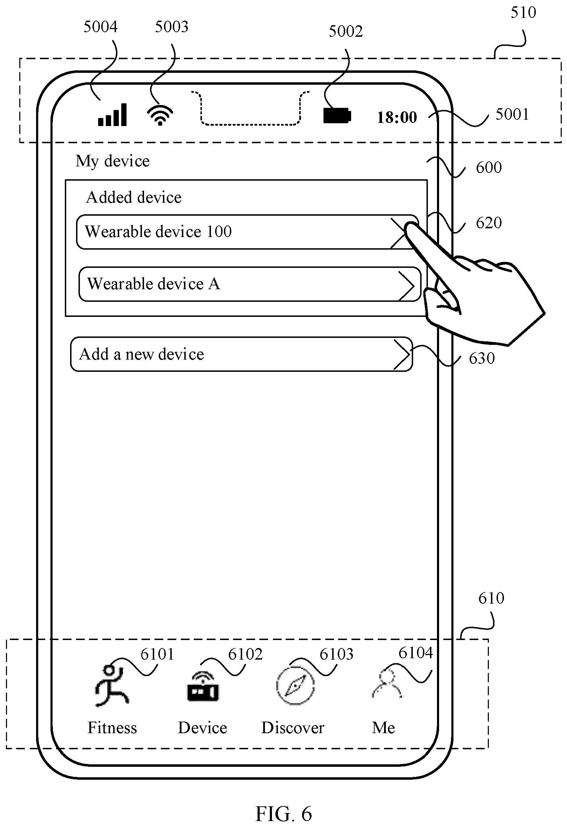

FIG. 6 is a diagram of an interface of a healthy application according to an embodiment of this application;

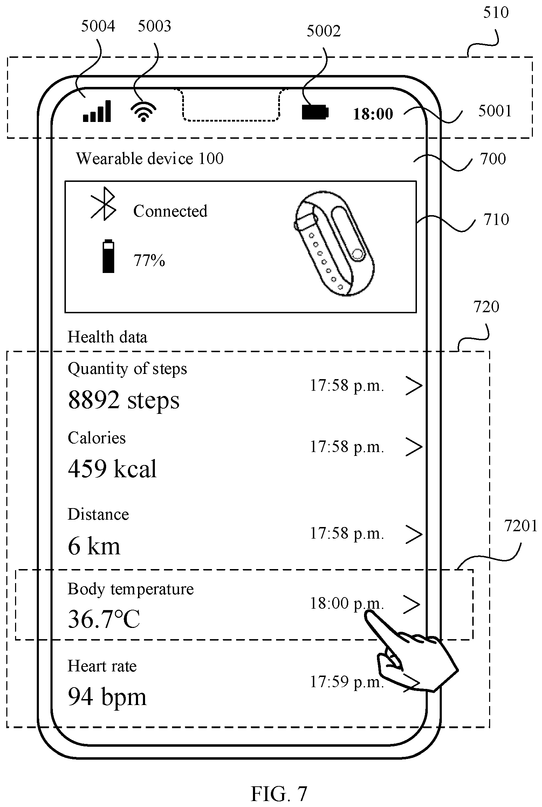

FIG. 7 is a diagram of an application interface according to an embodiment of this application;

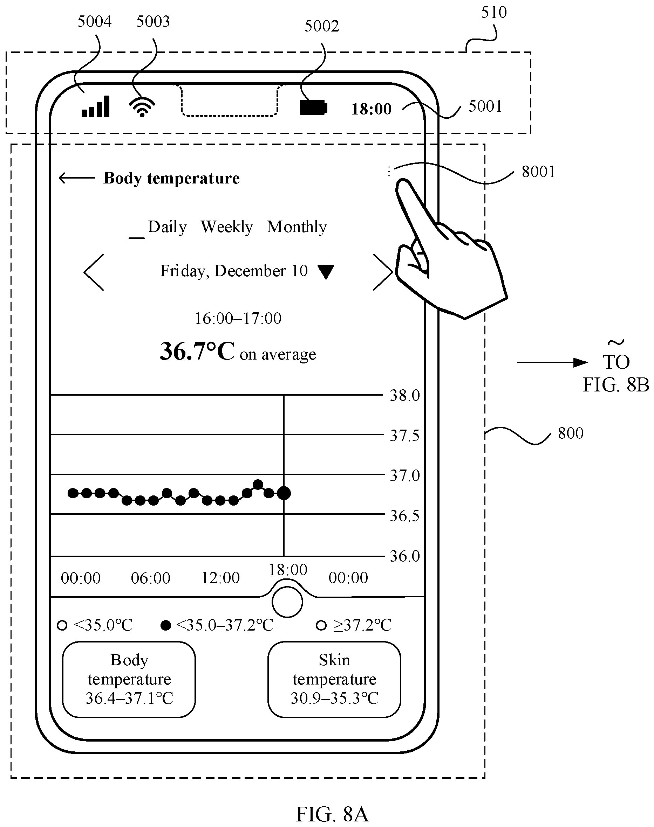

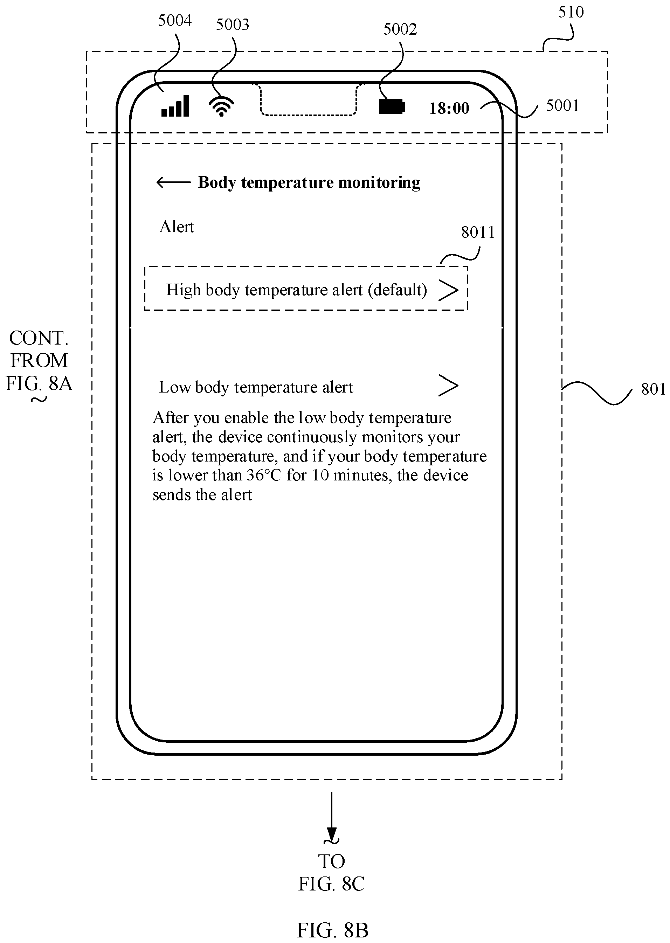

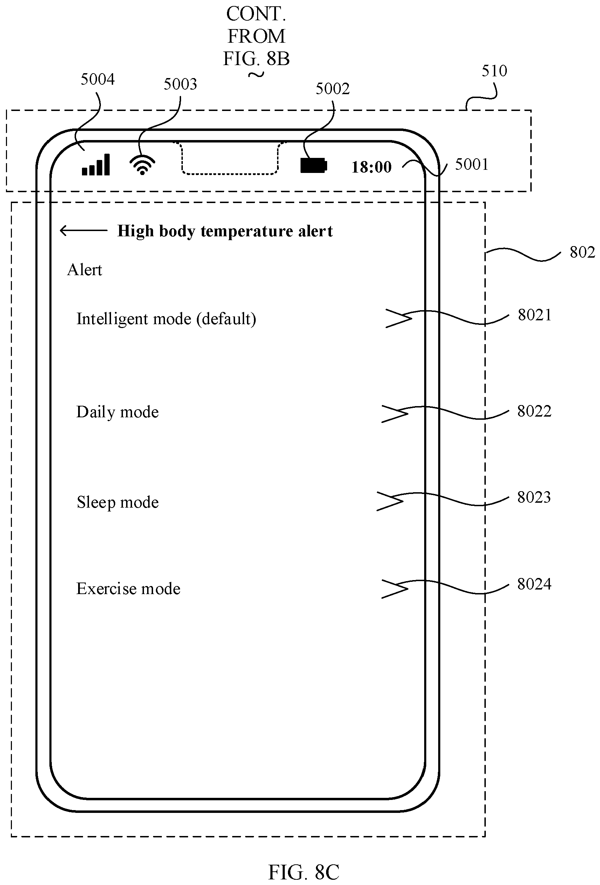

FIG. 8A to FIG. 8C are a diagram of a body temperature user interface according to an embodiment of this application;

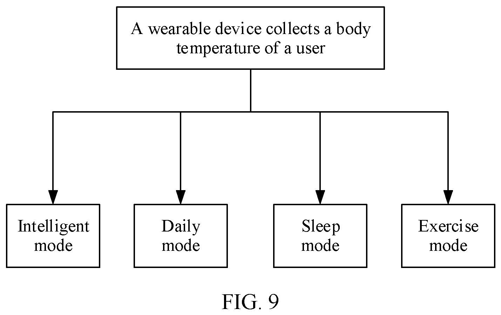

FIG. 9 is a diagram of body temperature monitoring modes according to an embodiment of this application;

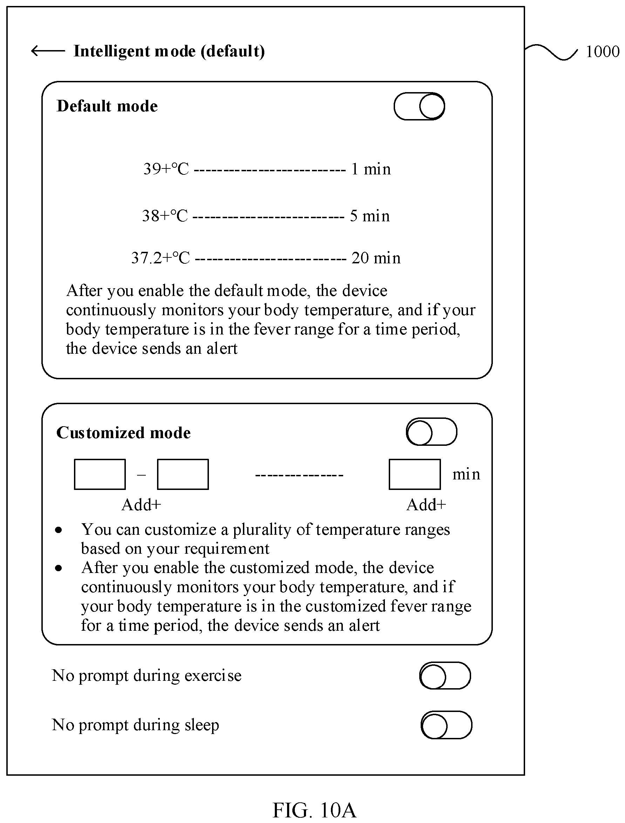

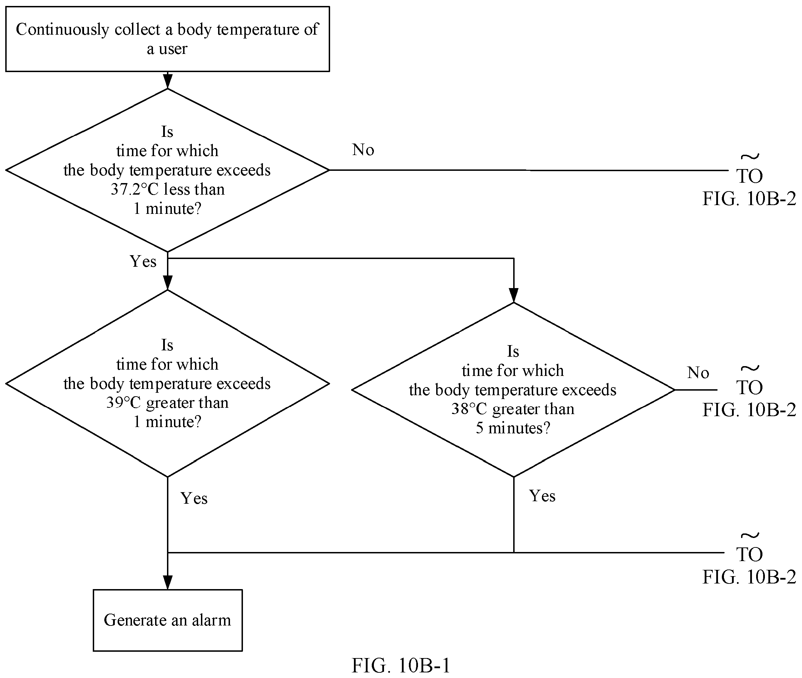

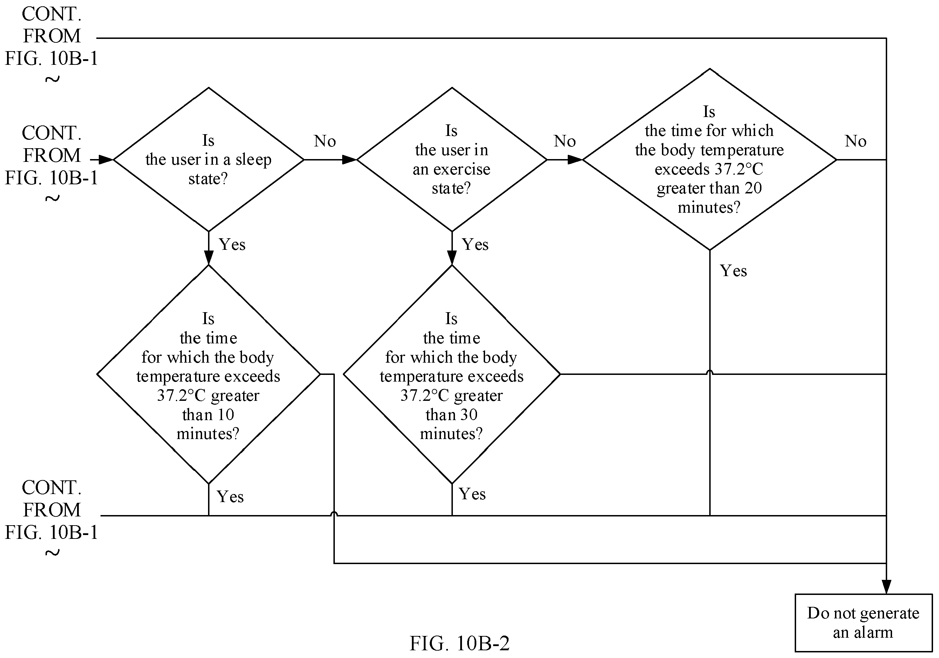

FIG. 10A is a diagram of a user interface of an intelligent mode according to an embodiment of this application;
FIG. 10B-1 and FIG. 10B-2 are a schematic flowchart of a fever alarm in an intelligent mode according to an embodiment of this application;

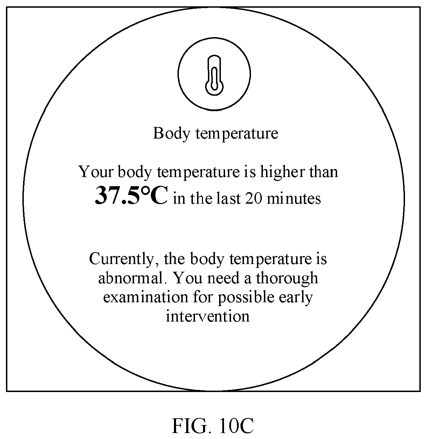

FIG. 10C is a diagram of a user interface for generating a fever alarm by a wearable device in an intelligent mode according to an embodiment of this application;

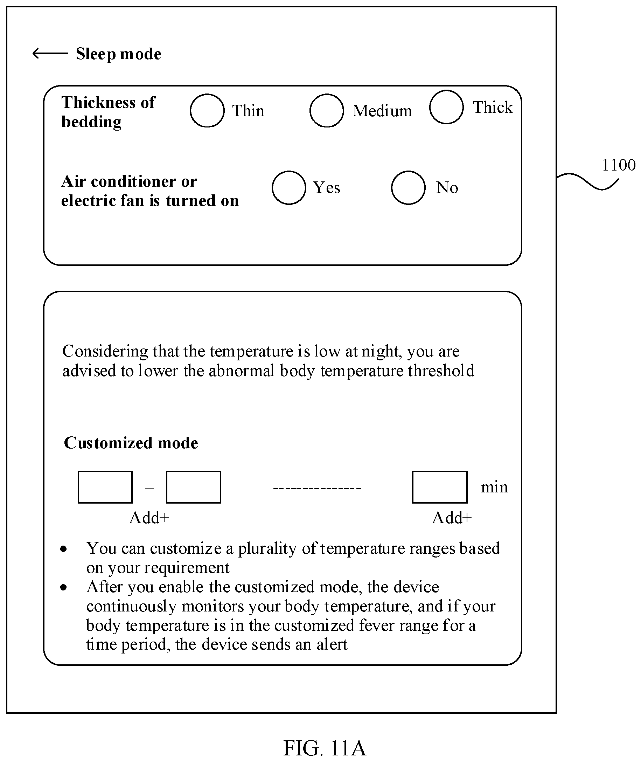

FIG. 11A is a diagram of a user interface of a sleep mode according to an embodiment of this application;

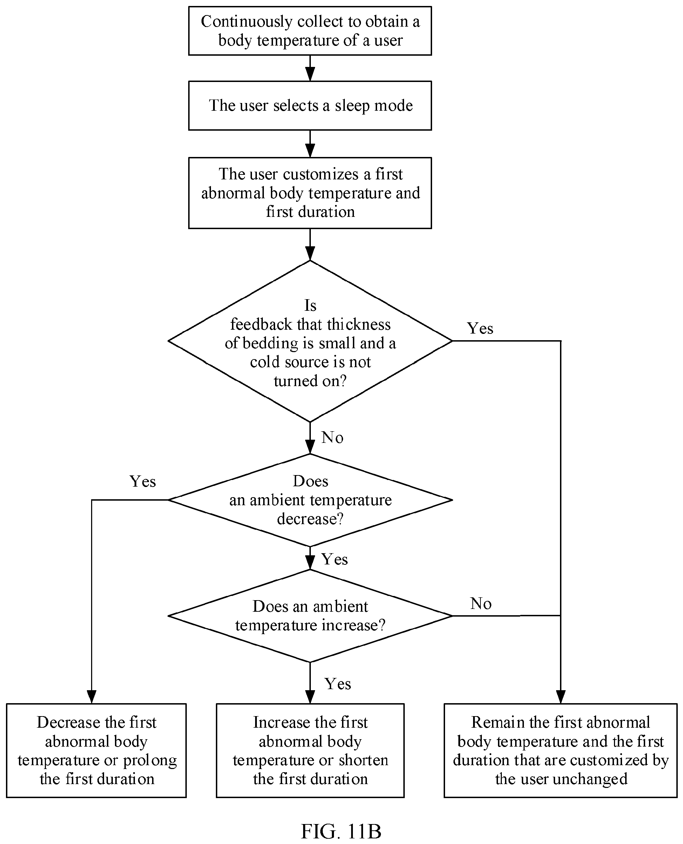

FIG. 11B is a schematic flowchart of a fever alarm in a sleep mode according to an embodiment of this application;

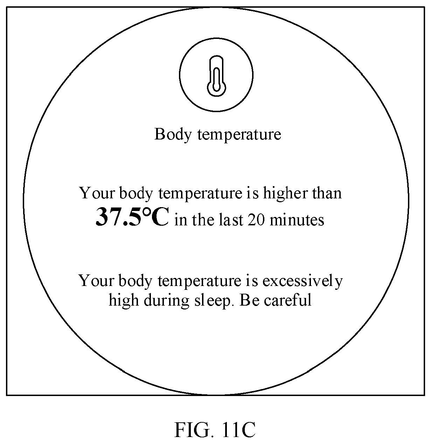

FIG. 11C is a diagram of a user interface for generating a fever alarm by a wearable device in a sleep mode according to an embodiment of this application;

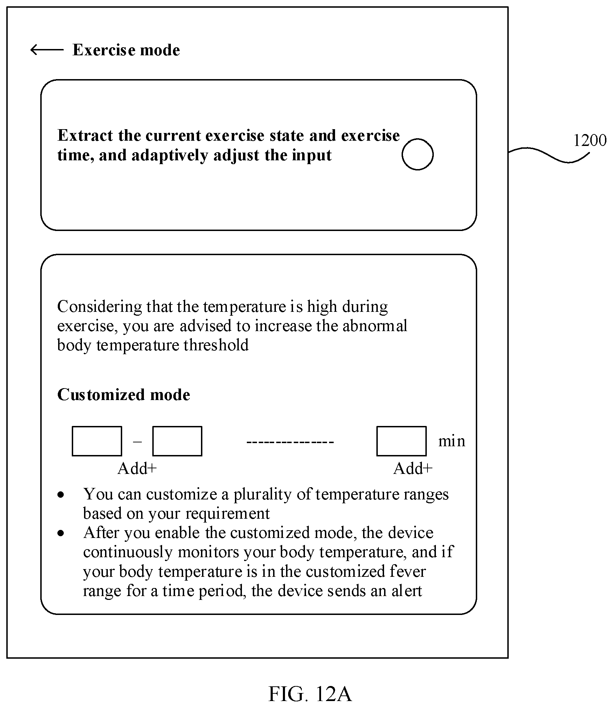

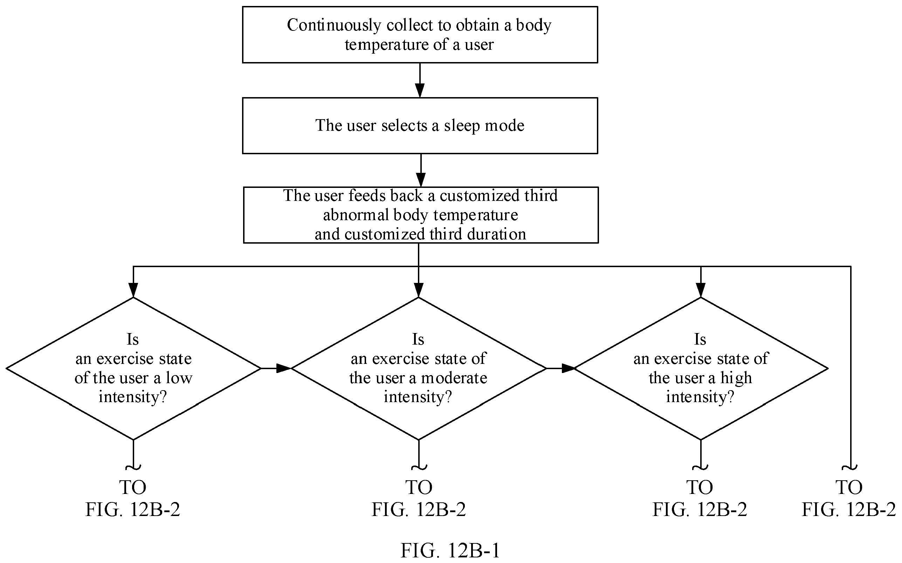

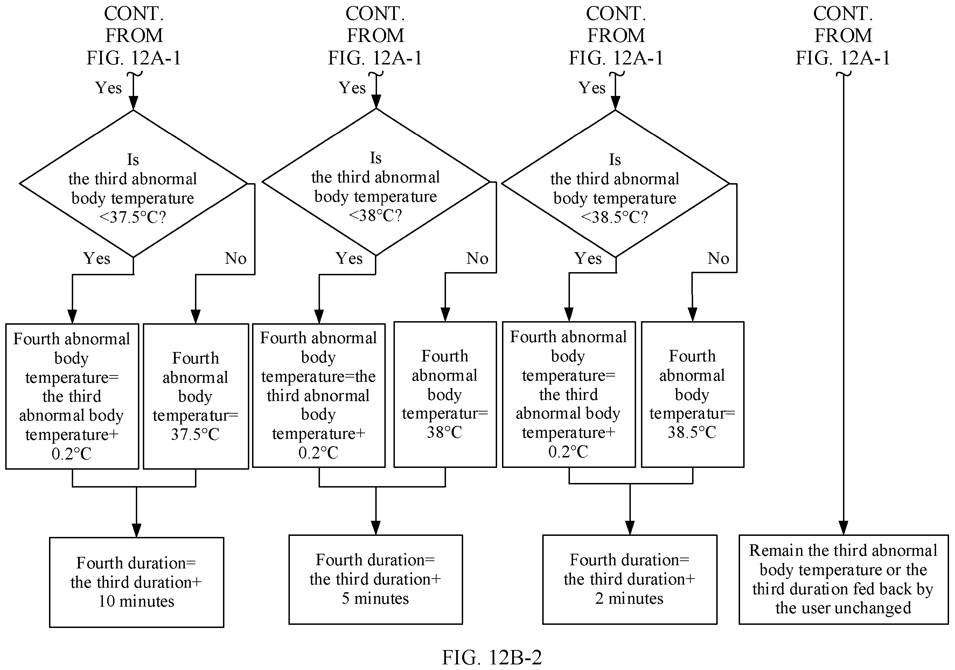

FIG. 12A is a diagram of a user interface of an exercise mode according to an embodiment of this application;
FIG. 12B-1 and FIG. 12B-2 are a schematic flowchart of a fever alarm in an exercise mode according to an embodiment of this application;

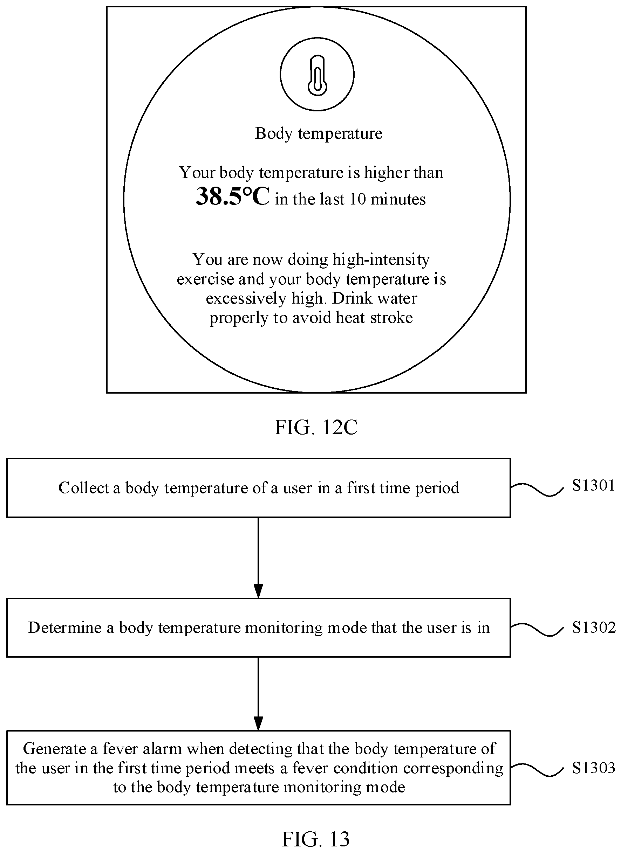

FIG. 12C is a diagram of a user interface for generating a fever alarm by a wearable device in an exercise mode according to an embodiment of this application; and
FIG. 13 is a schematic flowchart of a fever alarm method based on body temperature monitoring according to an embodiment of this application.

### DESCRIPTION OF EMBODIMENTS

The following clearly describes technical solutions in embodiments of this application in detail with reference to accompanying drawings. In descriptions of embodiments of this application, unless otherwise specified, "/" means "or". For example, A/B may indicate A or B. A term "or" in this specification is merely an association relationship for describing associated objects, and indicates that three relationships may exist. For example, A or B may indicate the following three cases: Only A exists, both A and B exist, and only B exists. In addition, in the descriptions of embodiments of this application, "a plurality of" means two or more.

Terms "first" and "second" below are merely intended for description, and shall not be understood as an implication or implication of relative importance or implicit indication of a quantity of indicated technical features. Therefore, a feature limited by "first" or "second" may explicitly or implicitly include one or more features. In the descriptions of embodiments of this application, unless otherwise specified, "a plurality of" means two or more.

A term "user interface (user interface, UI)" in the specification, claims, and accompanying drawings of this application is a medium interface for interaction and information exchange between a user and an application or an operating system, and implements conversion between an internal form of information and a form acceptable to the user. A user interface of an application is source code written through a specific computer language like Java or an extensible markup language (extensible markup language, XML). The interface source code is parsed and rendered on a terminal device, and finally displayed as user-recognizable content, for example, a control like a picture, text, or a button. A control (control) is also referred to as a widget (widget), and is a basic element of a user interface. Typical controls include a toolbar (toolbar), a menu bar (menu bar), a text box (text box), a button (button), a scrollbar (scroll bar), a picture, and text. An attribute and content of a control on the interface are defined by a tag or a node. For example, a control included on an interface are defined in the XML by nodes such as <Textview>, <ImgView>, or <VideoView>. One node corresponds to one control or attribute on the interface. After being parsed and rendered, the node is displayed as user-visible content. In addition, interfaces of many applications such as a hybrid application (hybrid application) usually further include a web page. The web page, also referred to as a page, may be understood as a special control embedded on an application interface. The web page is source code written in a particular computer language, for example, a hypertext markup language (hypertext markup language, HTML), a cascading style sheets (cascading style sheets, CSS), or JavaScript (JavaScript, JS). The web page source code may be loaded and displayed as user-recognizable content by a browser or a web page display component with a function similar to a function of the browser. Specific content included in the web page is also defined by a tag or a node in the web page source code. For example, an element and an attribute of a web page are defined in the HTML by <p>, <img>, <video>, and <canvas>.

The user interface is usually represented in a form of a graphical user interface (graphical user interface, GUI), and is a user interface that is related to a computer operation and that is displayed in a graphical manner. The user interface may be an interface element like an icon, a window, or a control displayed on a display of an electronic device. The control may include a visual interface element like an icon, a button, a menu, a tab, a text box, a dialog box, a status bar, a navigation bar, or a widget.

Embodiments of this application provide a fever alarm method based on body temperature detection, and a wearable device (for example, a smart band, a smart watch, or a smart body temperature sticker) applied to a plurality of application scenarios for a fever alarm, covering daily home, office, sleep, exercise, and another scenario. A more accurate abnormal body temperature alarm can be provided through division into scenarios. In the four application scenarios and modes, a time dimension is introduced based on a fever segment threshold. Each body temperature segment corresponds to different duration, and the alarm is generated only when the duration is met.

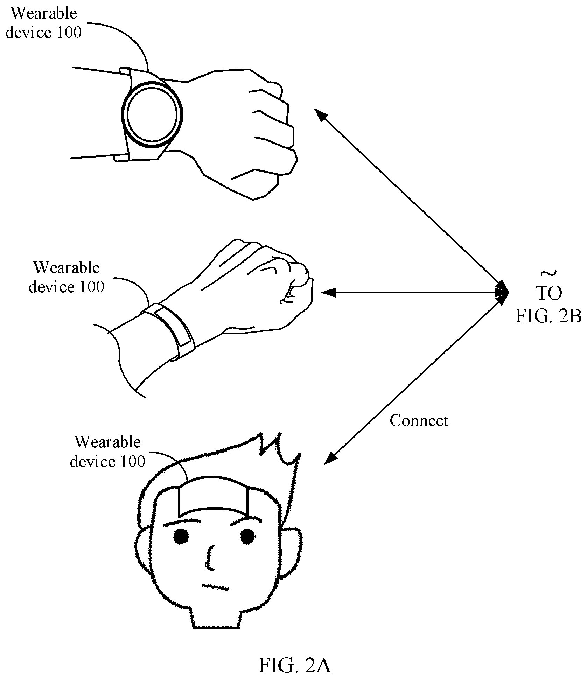

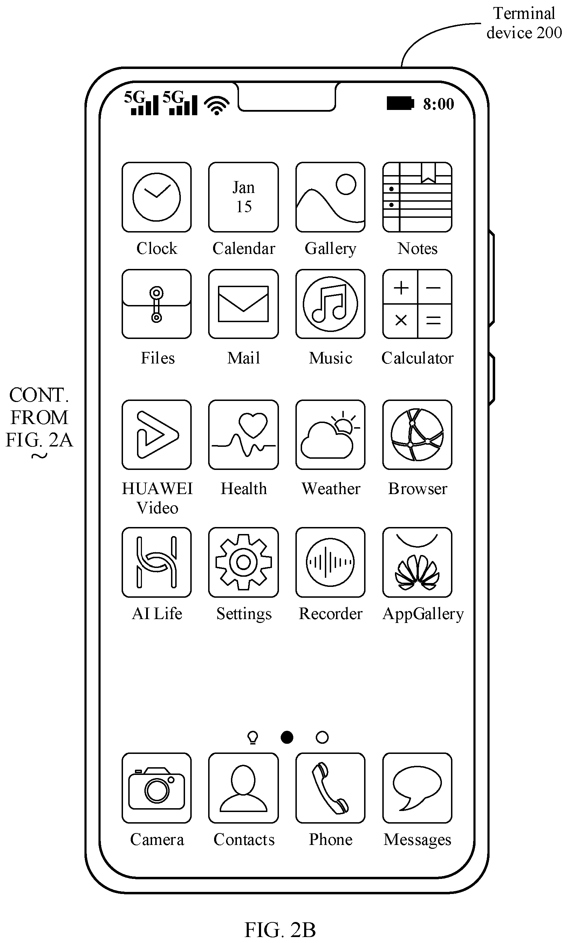

FIG. 2A and FIG. 2B are a diagram of a scenario of a fever alarm based on body temperature detection according to an embodiment of this application. It can be seen from FIG. 2A and FIG. 2B that wearable devices 100 and a terminal device 200 may be directly connected or communicated in a wired or wireless manner. In addition, the wearable devices 100 and the terminal device 200 may be connected to the internet in a wired or wireless manner, and communicate with each other over the internet. The wired manner includes, for example, a universal serial bus (universal serial bus, USB), a twisted pair, a coaxial cable, and an optical fiber. The wireless manner includes, for example, wireless fidelity (wireless fidelity, Wi-Fi), Bluetooth, and cellular communication. The internet includes, for example, at least one server. The server may be a hardware server, or may be a cloud server.

The wearable device 100 provided in this embodiment of this application is a portable device that may be attached to skin of a user. When the wearable device 100 is a smart watch or a smart band, the wearable device 100 may be attached to a wrist part of the user. This facilitates collection of a body temperature of the user. When the wearable device 100 is a smart body temperature sticker, the wearable device 100 may be attached to a skin surface like a forehead or an armpit of the user. This facilitates collection of a body temperature of the user.

The terminal device 200 may be a smart screen, a smartphone, a mobile phone, a tablet computer, a desktop computer, a laptop computer, a notebook computer, an ultra-mobile personal computer (Ultra-mobile Personal Computer, UMPC), a handheld computer, a netbook, a personal digital assistant (Personal Digital Assistant, PDA), or the like.

In some embodiments, the wearable device 100 may enable a body temperature detection mode by monitoring a user operation performed on the wearable device 100, to determine a body temperature detection mode that the user is in. The body temperature monitoring mode includes one or more of the following: an intelligent mode, a daily mode, a sleep mode, and an exercise mode.

In some other embodiments, after the wearable device 100 establishes a communication connection relationship with the terminal device 200, when detecting a user operation used to enable or disable the body temperature monitoring mode, the terminal device 200 may send, to the wearable device 100, an instruction for enabling the body temperature monitoring mode. When the wearable device 100 receives the instruction used to enable the body temperature monitoring mode, the wearable device 100 may determine the body temperature monitoring mode that the user is in.

When the wearable device 100 is attached to the skin surface of the user, the wearable device 100 may collect a body temperature of the user in a time period. When the wearable device 100 detects that the body temperature of the user in the time period meets fever time corresponding to the body temperature monitoring mode, the wearable device may display fever alarm information.

It may be understood that the wearable device 100 may send the fever alarm information to the terminal device 200. Therefore, the terminal device 200 may display the fever alarm information.

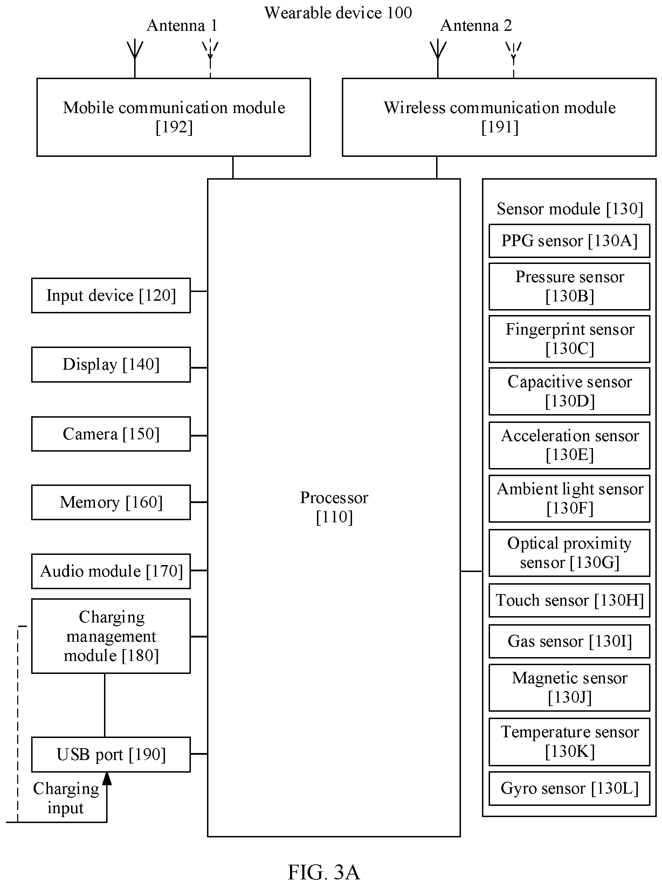

FIG. 3A is a schematic functional block diagram of a wearable device 100 according to an embodiment of this application. For example, the wearable device 100 may be a smart watch, a smart band, a smart body temperature sticker, or the like. The wearable device 100 may include a processor 110, an input device 120, a sensor module 130, a memory 160, an audio module 170, and a charging management module 180. It should be understood that the wearable device 100 shown in FIG. 3A is merely an example, and the wearable device 100 may have more or fewer components than those shown in FIG. 3A, may combine two or more components, or may have different component configurations. The components shown in the figure may be implemented by hardware including one or more signal processing and/or application-specific integrated circuits, software, or a combination of hardware and software.

The processor 110 may include one or more processing units. For example, the processor 110 may include an application processor (application processor, AP), a modem processor, a graphics processing unit (graphics processing unit, GPU), an image signal processor (image signal processor, ISP), a controller, a memory, a video codec, a digital signal processor (digital signal processor, DSP), a baseband processor, a neural-network processing unit (neural-network processing unit, NPU), or the like. Different processing units may be independent components, or may be integrated into one or more processors. The controller may be a nerve center and a command center of the wearable device 100. The controller may generate an operation control signal based on an instruction operation code and a time sequence signal, to complete control of instruction fetching and instruction execution. A memory may be further disposed in the processor 110, and is configured to store instructions and data. In some embodiments, the memory in the processor 110 is a cache. The memory may store instructions or data that has been used or cyclically used by the processor 110. If the processor 110 needs to use the instructions or the data again, the processor 110 may directly invoke the instructions or the data from the memory. This avoids repeated access, reduces waiting time of the processor 110, and improves efficiency of the wearable device 100.

The input device 120 is configured to provide a user input, and may be a mechanical device. A user touches the input device 120, so that the input device 120 rotates, translates, or tilts to implement a user input, to implement functions or operations such as starting (for example, powering on or off), or determining or adjusting information (for example, adjusting a volume) of the wearable device 100.

It may be understood that the input device 120 may be one component or a combination of a plurality of components. In some embodiments, the input device 120 may include a button, and the button may implement a rotary input. For example, the button may further implement a movement input like tilting or translation. In an embodiment in which the wearable device 100 is the smart watch, the button may also be referred to as a watch crown. In some other embodiments, the input device 120 may include a key. The key may implement a movement input like tilting or pressing. For example, the key may be a power key or a volume key of the wearable device 100. In some other embodiments, the input device 120 may include a button and a key. In some other embodiments, the wearable device 100 may further include a housing (not shown in FIG. 3A), and the input device 120 may be attached to an outer side of the housing and extend to the inside of the housing. The housing may be made of various materials, including but not limited to, plastic, metal, alloy, and the like.

It may be further understood that the wearable device 100 may include one or more input devices 120.

In some other embodiments, the input device 120 may include implementing a plurality of functions. For example, the input device 120 may implement functions such as fingerprint recognition, photoplethysmography (photoplethysmography, PPG) detection, photographing, gas detection, ambient light detection, body temperature detection, and light-emitting lighting.

The sensor module 130 may include one or more sensors, for example, may include a PPG sensor 130A, a pressure sensor 130B, a fingerprint sensor 130C, a capacitive sensor 130D, an acceleration sensor 130E, an ambient light sensor 130F, an optical proximity sensor 130G, a touch sensor 130H, a gas sensor 130I, a magnetic sensor 130J, and a temperature sensor 130K. It should be understood that FIG. 3A shows only an example in which several types of sensors are listed. In actual application, the wearable device 100 may further include more or fewer sensors, replace the listed sensor with another sensor with a same or similar function, or the like. This is not limited in embodiments of this application.

In some embodiments, the sensor module 130 may detect a user input from the input device 120, and respond to the user input to enable, determine, or adjust a signal light function or operation.

The PPG sensor 130A may be configured to detect a heart rate, that is, a quantity of heartbeats per unit time. In some embodiments, the PPG sensor 130A may include an optical sending unit and an optical receiving unit. The optical sending unit may irradiate a light beam into a human body (for example, a blood vessel), the light beam is reflected/refracted in the human body, and reflected/refracted light is received by the optical receiving unit, to obtain an optical signal. Because transmittance changes in a blood fluctuation process, the reflected/refracted light changes, and an optical signal detected by the PPG sensor 130A also changes. The PPG sensor 130A may convert the optical signal into an electrical signal, to determine a heart rate corresponding to the electrical signal. In this embodiment of this application, the PPG sensor 130A may be disposed in the input device 120 or disposed in the housing, and a function of PPG detection may be implemented by using an optical signal detected by the PPG sensor 130A.

The pressure sensor 130B may be configured to detect a pressure value between a human body and the wearable device 100. The pressure sensor 130B is configured to sense a pressure signal, and may convert the pressure signal into an electrical signal. There are many types of pressure sensors 130B, such as a resistive pressure sensor, an inductive pressure sensor, and a capacitive pressure sensor. This is not limited in embodiments of this application. In this embodiment of this application, a plurality of pressure sensors 130B may be disposed on the input device 120, and rotation of the input device 120 is identified by using a signal difference between adjacent pressure sensors 130B in the plurality of pressure sensors 130B.

The fingerprint sensor 130C is configured to collect a fingerprint. The wearable device 100 may use a feature of the collected fingerprint to implement fingerprint-based unlocking, application lock access, fingerprint-based photographing, fingerprint-based call answering, and the like. There are many types of fingerprint sensors 130C. For example, the fingerprint sensor 130C may include an optical fingerprint sensor, a semiconductor fingerprint sensor, and an ultrasonic sensor. The semiconductor fingerprint sensor may include a capacitive sensor, a thermosensitive sensor, a pressure-sensitive sensor, and the like. In this embodiment of this application, the fingerprint sensor 130C may be disposed in the input device 120 or disposed in the housing, and a function of fingerprint recognition may be implemented by using light reflected by the input device 120.

The capacitive sensor 130D may be configured to detect a capacitance between two electrodes, to implement a specific function.

In some embodiments, the capacitive sensor 130D may be configured to detect a capacitance between a human body and the wearable device 100. The capacitance may reflect whether the human body is in good contact with the wearable device 100, and may be used in electrocardiography (electrocardiography, ECG) detection. The human body can be used as an electrode. When the capacitive sensor 130D is disposed on an electrode of the wearable device 100, the capacitive sensor 130D may detect a capacitance between the human body and the electrode. When the capacitance detected by the capacitive sensor 130D is too large or too small, it indicates that contact between the human body and the electrode is poor. When the capacitance detected by the capacitive sensor 130D is moderate, it indicates that the human body is in good contact with the electrode. Whether the human body is in good contact with the electrode affects detection of an electrical signal by the electrode, and further affects ECG generation. Therefore, when generating the ECG, the wearable device 100 may refer to the capacitance detected by the capacitive sensor 130D.

In some other embodiments, the capacitive sensor 130D may generate a varying capacitance between the capacitive sensor 130D and a metal electrode disposed in the input device 120, and identify rotation or movement of the input device 120 based on the varying capacitance.

The acceleration sensor 130E may be configured to detect magnitudes of accelerations of the wearable device 100 in all directions (usually on three axes). When the user wears the wearable device 100, the wearable device 100 moves under driving of the user. Therefore, the magnitudes of the accelerations detected by the acceleration sensor 130E in all the directions may reflect a movement status of the human body.

The ambient light sensor 130F is configured to sense an ambient light parameter. For example, the ambient light parameter may include an ambient light intensity, a coefficient of an ultraviolet ray in the ambient light, or the like. The wearable device 100 may adaptively adjust brightness of a display based on the sensed ambient light intensity. The ambient light sensor 130F may also be configured to automatically adjust white balance during photographing. The ambient light sensor 130F may further cooperate with the optical proximity sensor 130G to detect whether the wearable device 100 is in a pocket, to avoid an accidental touch. In this embodiment of this application, the ambient light sensor 130F may be disposed in the input device 120 or disposed in the housing, and a function of ambient light detection may be implemented by detecting, by using the ambient light sensor 130F, an ambient light parameter in an environment in which the wearable device 100 is located.

The optical proximity sensor 130G may include, for example, a light-emitting diode (LED) and an optical detector, for example, a photodiode. The light-emitting diode may be an infrared light-emitting diode. The wearable device 100 emits infrared light by using the light-emitting diode. The wearable device 100 detects infrared reflected light from a nearby object by using the photodiode. When sufficient reflected light is detected, it may be determined that there is an object near the wearable device 100. When insufficient reflected light is detected, the wearable device 100 may determine that there is no object near the wearable device 100. The wearable device 100 may detect, by using the optical proximity sensor 130G, that the user holds the wearable device 100 close to an ear for a call, to automatically turn off a screen for power saving. The optical proximity sensor 130G may also be used in a smart cover mode or a pocket mode to automatically perform screen unlocking or locking.

The touch sensor 130H may be disposed on a display, and the touch sensor 130H and the display form a touchscreen, which is also referred to as a "touchscreen". The touch sensor 130H is configured to detect a touch operation performed on or near the touch sensor 130H. The touch sensor 130H may transfer the detected touch operation to the processor, to determine a type of a touch event. Visual output related to the touch operation may be provided on a display. In some other embodiments, the touch sensor 130H may alternatively be disposed on a surface of a display, and is in a position different from that of the display.

The gas sensor 130I is configured to detect a gas parameter. For example, the gas parameter may include a gas type, a gas concentration, or the like. In this embodiment of this application, the gas sensor 130I may be disposed in the input device 120 or disposed in the housing, and a function of gas detection may be implemented by detecting, by using the gas sensor 130I, a gas parameter in an environment in which the wearable device 100 is located.

The magnetic sensor 130J is a device that detects corresponding physical quantities by converting a magnetic property change of a sensitive element caused by external factors such as a magnetic field, a current, stress and strain, a temperature, and light into electrical signals. In this embodiment of this application, the magnetic sensor 130J may be combined with a magnetic layer in the input device 120. When the input device 120 is rotated, changed flux is generated. Rotation or movement of the input device 120 is identified by using an induced current generated on the magnetic sensor 130J.

The temperature sensor 130K is configured to collect a temperature. In some embodiments, the collected temperature may be a human body temperature or an ambient temperature. In some embodiments, the wearable device 100 executes a temperature processing strategy based on the temperature detected by the temperature sensor 130K. For example, when the temperature reported by the temperature sensor 130K exceeds a threshold, the wearable device 100 lowers performance of a processor nearby the temperature sensor 130K, to reduce power consumption to implement thermal protection.

A gyro sensor 130L may be configured to determine a movement posture of the wearable device 100. In some embodiments, an angular velocity of the wearable device 100 around three axes (namely, axes x, y, and z) may be determined by using the gyro sensor 130L.

The memory 160 may be configured to store computer-executable program code. The executable program code includes instructions. The processor 110 runs the instructions stored in the memory 160, to implement various function applications of the wearable device 100 and data processing. The memory 160 may include a high-speed random access memory, and may further include a non-volatile memory, for example, at least one magnetic disk storage device, a flash storage device, or a universal flash storage (universal flash storage, UFS). This is not limited in embodiments of this application.

The charging management module 180 may be configured to supply power to components, such as the processor 110 and the sensor module 130 in the wearable device 100. In some embodiments, the charging management module 180 may be a battery or another portable power component. In some other embodiments, the wearable device 100 may further be connected to a charging device (for example, through a wireless connection or a wired connection), and the charging management module 180 may receive electric energy input by the charging device, to store electricity in a battery.

In some embodiments, still refer to FIG. 3A. The wearable device 100 further includes a display 140. The display 140 includes a display panel. The display panel may be a liquid crystal display (liquid crystal display, LCD), an organic light-emitting diode (organic light-emitting diode, OLED), an active-matrix organic light-emitting diode (active-matrix organic light-emitting diode, AMOLED), a flexible light-emitting diode (flexible light-emitting diode, FLED), a mini-LED, a micro-LED, a micro-OLED, a quantum dot light emitting diode (quantum dot light emitting diode, QLED), or the like. In some embodiments, a touch sensor may be disposed in the display to form a touchscreen. This is not limited in embodiments of this application.

It may be understood that, in some embodiments, the wearable device 100 may include the display 140, or may not include the display 140. For example, when the wearable device 100 is a band, the wearable device 100 may include a display or may not include a display. When the wearable device 100 is a watch, the wearable device 100 may include a display.

In some other embodiments, still refer to FIG. 3A. The wearable device 100 may further include a camera 150, configured to capture a static image or a video, and an optical image of an object is generated through the lens, and is projected onto a photosensitive element. The photosensitive element may be a charge-coupled device (charge-coupled device, CCD) or a complementary metal-oxide-semiconductor (complementary metal-oxide-semiconductor, CMOS) photoelectric transistor. The photosensitive element converts an optical signal into an electrical signal, and then transmits the electrical signal to the ISP to convert the electrical signal into a digital image signal. The ISP outputs the digital image signal to the DSP for processing. The DSP converts the digital image signal into an image signal in a standard format like RGB or YUV.

In some embodiments, the camera 150 may be applied to a front-facing photographing scenario, or may be referred to as a "front-facing camera" for short. In some other embodiments, the camera 150 is set to be rotatable, and may implement multi-directional or multi-angle photographing of a scene. For example, the camera 150 may be applied to a front-facing photographing scene or may be applied to a rear-facing photographing scene. In some other embodiments, the wearable device may include one or more cameras 150. This is not limited in this application. For example, the camera 150 has low resolution, a small volume, and occupies small device space. Therefore, the camera 150 may be well used in a wearable device whose volume is small and that is portable.

In some other embodiments, still refer to FIG. 3A. The wearable device 100 may further include the audio module 170, and the audio module 170 may include a device that can receive or output a sound signal, for example, a microphone, a loudspeaker, or an earpiece. The loudspeaker, also referred to as a "speaker", is configured to convert an audio electrical signal into a sound signal. The wearable device 100 may listen to music or answer a hands-free call by using the loudspeaker. The earpiece, also referred to as a "receiver", is configured to convert an audio electrical signal into a sound signal. When a call is answered or voice information is listened to by using the wearable device 100, voice may be listened to by placing the earpiece close to a human ear. The microphone, also referred to as a "mike" or a "mic", is configured to convert a sound signal into an electrical signal. When making a call or sending voice information, a user may make a sound near the microphone through the mouth of the user, to input the sound signal to the microphone. At least one microphone may be disposed in the wearable device 100.

In some other embodiments, two microphones may be disposed in the wearable device 100, to collect a sound signal and further implement a noise reduction function. In some other embodiments, three, four, or more microphones may alternatively be disposed in the wearable device 100, to collect a sound signal, reduce noise, identify a sound source, implement a directional recording function, and the like.

In addition, the wearable device 100 may have a wireless communication function. In some embodiments, still refer to FIG. 3A. The wearable device 100 may further include a wireless communication module 191, a mobile communication module 192, one or more antennas 1, and one or more antennas 2. The wearable device 100 may implement a wireless communication function by using the antenna 1, the antenna 2, the wireless communication module 191, and the mobile communication module 192.

In some embodiments, the wireless communication module 191 may provide a wireless communication solution that is applied to the wearable device 100 and that complies with various network communication protocols or communication technologies. For example, the network communication solution may include a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), a near field communication (near field communication, NFC) technology, an infrared (infrared, IR) technology, or the like. For example, the wearable device 100 may establish a Bluetooth connection to another electronic device like a mobile phone by using a Bluetooth protocol.

In some other embodiments, the wireless communication module 191 may be one or more components integrating at least one communication processor module. The wireless communication module 191 receives an electromagnetic wave by using the antenna 1, performs frequency modulation and filtering processing on an electromagnetic wave signal, and sends a processed signal to the processor 110. The wireless communication module 191 may further receive a to-be-sent signal from the processor 110, perform frequency modulation and amplification on the signal, and convert the signal into an electromagnetic wave by using the antenna 1 for radiation.

In some embodiments, the wireless communication module 191 may be coupled to one or more antennas 1, so that the wearable device 100 can communicate with a network and another device by using a wireless communication technology.

In some embodiments, the mobile communication module 192 may provide a wireless communication solution that is applied to the wearable device 100 and that complies with various network communication protocols or communication technologies. For example, the network communication protocol may be various wired or wireless communication protocols, such as the Ethernet, a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), voice over Internet protocol (voice over Internet protocol, VoIP), a communication protocol supporting a network slicing architecture, or any other suitable communication protocol. For example, the wearable device 100 may establish a wireless communication connection to another electronic device like a mobile phone by using a WCDMA communication protocol.

In some other embodiments, the mobile communication module 192 may include at least one filter, a switch, a power amplifier, a low noise amplifier (low noise amplifier, LNA), and the like.

In some other embodiments, at least some functional modules of the mobile communication module 192 may be disposed in the processor 110.

In some other embodiments, at least some functional modules of the mobile communication module 192 may be disposed in a same device as at least some modules of the processor 110. The mobile communication module 192 may receive an electromagnetic wave by using the antenna 2, perform processing such as filtering or amplification on the received electromagnetic wave, and transmit the electromagnetic wave to the modem processor for demodulation. The mobile communication module 192 may further amplify a signal modulated by the modem processor, and convert the signal into an electromagnetic wave by using the antenna 2 for radiation.

In some embodiments, the mobile communication module 192 may be coupled to one or more antennas 2, so that the wearable device 100 can communicate with a network and another device by using a wireless communication technology.

A software system of the wearable device 100 may use a layered architecture, an event-driven architecture, a microkernel architecture, a micro service architecture, or a cloud architecture. For example, a software system of a layered architecture may be an Android (Android) system, or may be a Huawei Mobile Services (Huawei Mobile Services, HMS) system. In addition, the software system of the wearable device 100 may alternatively be an iOS system. This is not limited in this application. In embodiments of this application, an Android system with a layered architecture is used as an example to describe the software structure of the wearable device 100.

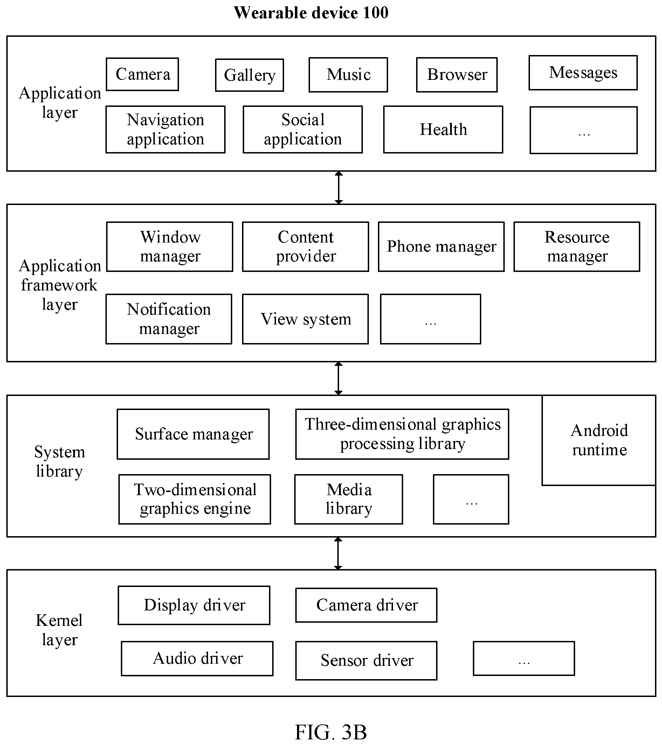

FIG. 3B is a diagram of the software architecture of the wearable device 100 according to this embodiment of this application.

In the layered architecture, software is divided into several layers, and each layer has a clear role and task. The layers communicate with each other through software interfaces. In some embodiments, the Android system is divided into four layers: which are respectively an application layer, an application framework layer, an Android runtime (Android runtime) and system library, and a kernel layer from top to bottom.

The application layer may include a series of application packages.

As shown in FIG. 3B, the application package may include applications such as Camera, Gallery, Music, Browser, Messages, a navigation application, a social application, and Health.

The application framework layer provides an application programming interface (application programming interface, API) and a programming framework for an application at the application layer. The application framework layer includes some predefined functions.

As shown in FIG. 3B, the application framework layer may include a window manager, a content provider, a view system, a phone manager, a resource manager, a notification manager, and the like.

The window manager is configured to manage a window program. The window manager may obtain a size of a display, determine whether there is a status bar, perform screen locking, take a screenshot, and the like.

The content provider is configured to: store and obtain data, and enable the data to be accessed by an application. The data may include a video, an image, an audio, calls that are made and answered, a browsing history and bookmarks, an address book, and the like.

The view system includes visual controls such as a control for displaying text and a control for displaying an image. The view system may be configured to construct an application. A display interface may include one or more views. For example, a display interface including an SMS message notification icon may include a text display view and an image display view.

The phone manager is configured to provide a communication function of the wearable device 100, for example, management of a call status (including answering, declining, or the like).

The resource manager provides various resources such as a localized character string, an icon, an image, a layout file, and a video file for an application.

The notification manager enables an application to display notification information in a status bar, and may be configured to convey a notification message. The notification manager may automatically disappear after a short pause without user interaction. For example, the notification manager is configured to notify download completion, give a message notification, and the like. The notification manager may alternatively be a notification that appears in a top status bar of the system in a form of a graph or a scroll bar text, for example, a notification of an application that is run on the background, or may be a notification that appears on the screen in a form of a dialog window. For example, text information is prompted in the status bar, a prompt tone is produced, the electronic device vibrates, or an indicator light blinks.

The Android runtime includes a core library and a virtual machine. The Android runtime is responsible for scheduling and management of the Android system.

The core library includes two parts: a performance function that needs to be invoked by Java language, and an Android core library.

The application layer and the application framework layer run on the virtual machine. The virtual machine executes Java files of the application layer and the application framework layer as binary files. The virtual machine is configured to implement functions such as object lifecycle management, stack management, thread management, security and exception management, and garbage collection.

The system library may include a plurality of functional modules, such as a surface manager (surface manager), a media library (Media Library), a three-dimensional graphics processing library (for example, OpenGL ES), and a 2D graphics engine (for example, SGL).

The surface manager is configured to: manage a display subsystem, and provide fusion of 2D and 3D layers for a plurality of applications.

The media library supports playback and recording of audio and videos in a plurality of commonly used formats, static image files, and the like. The media library may support a plurality of audio and video coding formats, for example, MPEG 4, H.264, MP3, AAC, AMR, JPG, and PNG.

The three-dimensional graphics processing library is configured to implement three-dimensional graphics drawing, image rendering, composition, layer processing, and the like.

The 2D graphics engine is a drawing engine for 2D drawing.

The kernel layer is a layer between hardware and software. The kernel layer includes at least a display driver, a camera driver, an audio driver, and a sensor driver.

The following describes an example of a working procedure of software and hardware of the wearable device 100 with reference to a process in which a user manually operates the wearable device 100 to obtain an unread message.

When the touch sensor 130H in FIG. 3A receives a touch operation, a corresponding hardware interruption is sent to the kernel layer. The kernel layer processes the touch operation into an original input event (including information such as touch coordinates and a time stamp of the touch operation). The original input event is stored at the kernel layer. The application framework layer obtains the original input event from the kernel layer, and identifies a control corresponding to the input event. For example, the touch operation is a single-tap touch operation, and a control corresponding to the single-tap operation is a notification control of a social application in a notification bar. The social application invokes an interface of the application framework layer to start the social application, then enables the display driver by invoking the kernel layer, and displays detailed information of the unread message corresponding to the notification control on the display 140 shown in FIG. 3A.

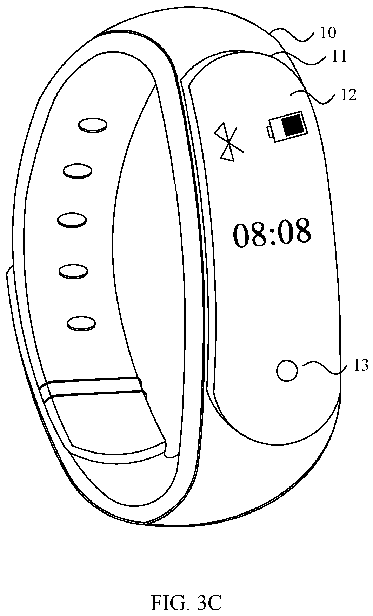

The following describes the wearable device by using a smart band as an example. FIG. 3C is a diagram of a structure of a wearable device 100. The wearable device 100 may include a device main body 11 and a wearable component 10. Details are as follows:

The wearable component 10 may be fastened or movably connected to the device main body 11, and the wearable component 10 may be wound around a wrist, an arm, a leg, or another part of a body, to fasten the wearable device 100 to a user.

An acceleration sensor, a gyro sensor, a sound collector, and a temperature sensor are disposed in the device main body 11. The device main body 11 may separately collect acceleration data, angular velocity data, a sound signal, a shell temperature, and an ambient temperature by using the acceleration sensor, the gyro sensor, the sound collector, and the temperature sensor. The device main body 11 may include a display 12 and a touch control 13. The display may be configured to display time, a battery level, a Bluetooth identifier, a received message of the device main body 11, and the like. The touch control 13 may be used to receive an operation of lighting up the display by the user, display, and receive a body temperature monitoring mode.

The device main body 11 may further record wearing time of the user, a collected shell temperature and ambient temperature, and calories burnt, and has a basic function like message notification.

The device main body 11 may establish a wireless communication connection to a terminal device 200 (for example, a mobile phone or a tablet computer).

In a possible implementation, the device main body 11 may establish a wireless communication connection to the terminal device 200 through Bluetooth. The device main body 11 may send the shell temperature of the user, the ambient temperature, exercise data of the user, and the like to the terminal device 200 to which the device main body 11 establishes the connection.

The wearable component 10 is configured to mount the device main body 11. For example, the wearable component 10 may be an apparatus like a wristband or a watchband. The wearable component 10 is in a state in which the device main body can be attached to the wrist of the user.

It may be understood that FIG. 3C is the diagram of the structure of the wearable device 100 when the wearable device 100 is the smart band. When the wearable device 100 is a smart watch or a smart body temperature sticker, the wearable device 100 has a structure the same as or similar to that of the smart band.

When the wearable device 100 enables the body temperature monitoring mode, the wearable device may collect body temperature data of the user, so that body temperatures of the user in different modes can be monitored, and when a detected body temperature meets a fever condition corresponding to the mode, a fever alarm is generated. The wearable device 100 may receive an input of the user, to enable the body temperature monitoring mode.

The wearable device 100 receives and responds to the input of the user, and enables the body temperature monitoring mode.

In some embodiments, the wearable device 100 may enable the body temperature monitoring mode by monitoring a user operation performed on the touch control 13.

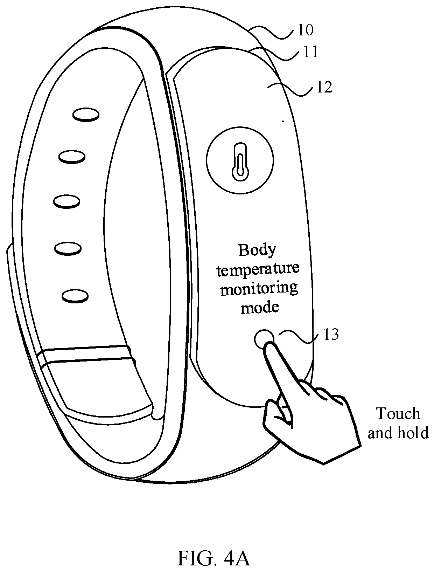

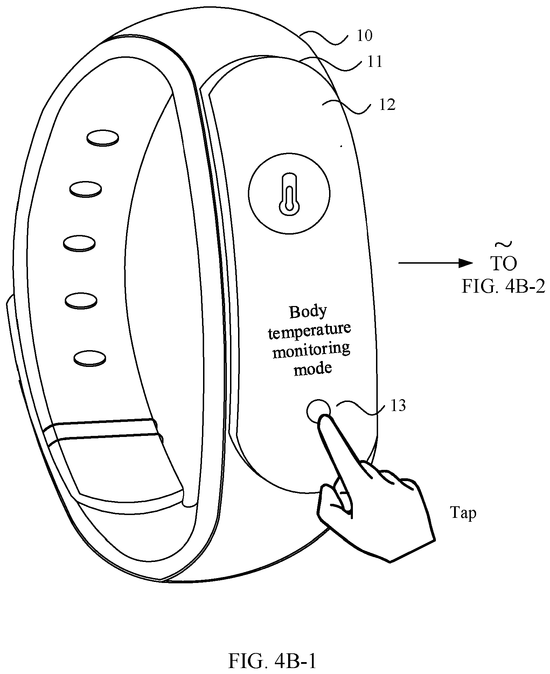

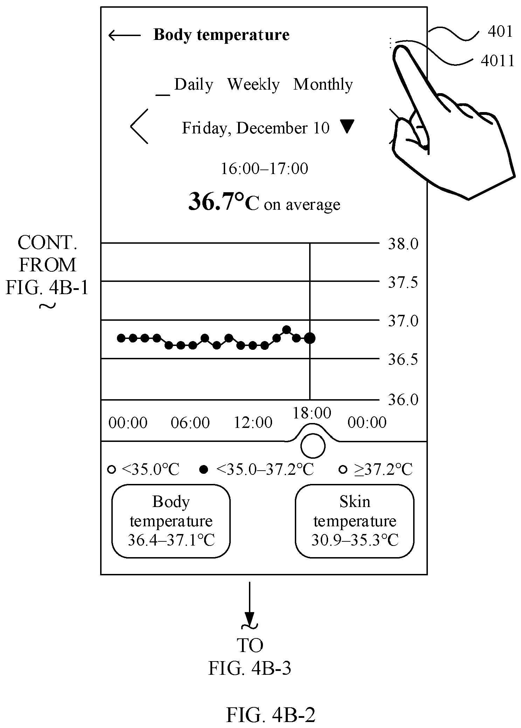

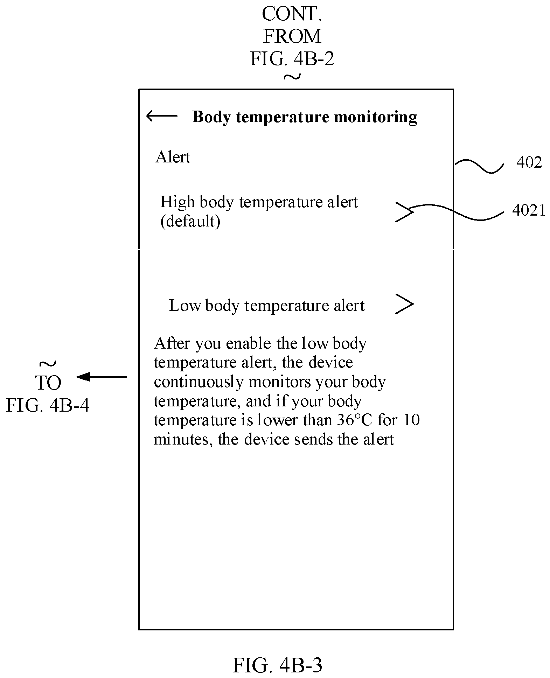

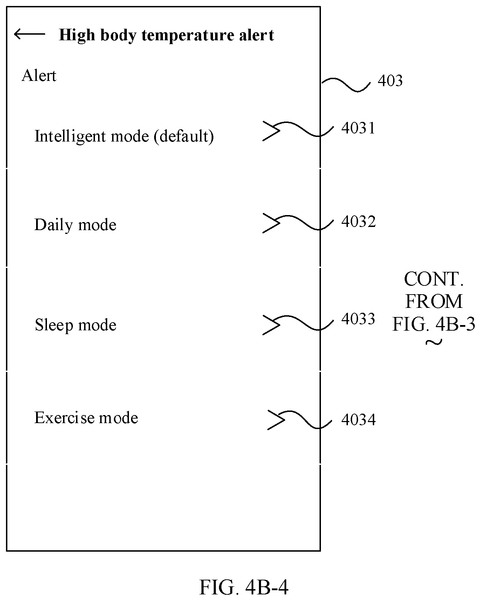

For example, as shown in FIG. 4A, when the wearable device 100 detects a touch-and-hold operation performed on the touch control 13, the wearable device 100 may enable or disable the body temperature monitoring mode. The wearable device 100 may enable the body temperature monitoring mode after countdown of specific time (for example, 3 seconds) after vibration. After the body temperature monitoring mode is enabled, the wearable device 100 may display a body temperature icon and text "Body temperature monitoring mode" on the display 12. In this way, the user may be prompted that the wearable device 100 has enabled the body temperature monitoring mode. It should be noted that the body temperature monitoring mode enabled through the touch- and-hold operation is a default mode of a system, namely, a high body temperature alert in an intelligent mode. When detecting that the collected body temperature of the user meets fever time corresponding to the intelligent mode, the wearable device 100 displays fever alarm information.

For another example, as shown in FIG. 4B-1 to FIG. 4B-4, when the wearable device 100 detects a tap operation performed on the touch control 13, the wearable device 100 may display a user interface 401. The user interface 401 may display body temperatures of the user collected by the wearable device 100, for example, body temperatures and skin temperatures collected in time for which the wearable device is worn, an average temperature of the user at current time, and the like.

When the wearable device 100 detects an operation performed on an option 4011 on the user interface 401, the wearable device 100 may display a user interface 402. Two alert modes provided by the wearable device 100 may be displayed on the user interface 402, namely, a high body temperature alert and a low body temperature alert respectively. For example, the high body temperature alert is a default alert manner of the system, and the user may change a default alert manner of the system based on an actual requirement of the user.

When the wearable device 100 detects an operation performed on an option 4021 on the user interface 402, the wearable device 100 may display a user interface 403. Four body temperature monitoring modes provided by the wearable device 100 may be displayed on the user interface 403: which are respectively an intelligent mode, a daily mode, a sleep mode, and an exercise mode. The intelligent mode is a default mode of the system, and the user may determine, based on an actual requirement of the user, a body temperature monitoring mode that the user is in.

In a possible implementation, if the user selects the intelligent mode in the body temperature monitoring mode, the user may customize and adjust, by using an intelligent mode option 4031, a setting related to a fever alarm in the intelligent mode. If the user selects the daily mode in the body temperature monitoring mode, the user may customize and adjust, by using a daily mode option 4032, a setting related to a fever alarm in the daily mode. If the user selects the sleep mode in the body temperature monitoring mode, the user may customize and adjust, by using a sleep mode option 4033, a setting related to a fever alarm in the sleep mode. If the user selects the exercise mode in the body temperature monitoring mode, the user may customize and adjust, by using an exercise mode option 4034, a setting related to a fever alarm in the exercise mode.

In a possible implementation, the wearable device 100 automatically turns off the display 12 after enabling the body temperature monitoring mode for a time period, so that power consumption of the wearable device 100 can be effectively reduced.

In some embodiments, after the wearable device 100 establishes the communication connection to the terminal device 200, the wearable device 100 may further receive a request for enabling the body temperature monitoring mode sent by the terminal device 200 to enable the body temperature monitoring mode.

A wearable device 100 may establish a communication connection relationship with a terminal device 200 (for example, a mobile phone or a tablet computer). When detecting a user operation of enabling or disabling a body temperature monitoring mode by a user, the terminal device 200 may send, to the wearable device 100, an instruction for enabling the body temperature monitoring mode. When the wearable device 100 receives the instruction used to enable the body temperature monitoring mode, the wearable device 100 may enable the body temperature monitoring mode.

As shown in FIG. 5, the terminal device 200 displays a user interface 500 on a home screen. The user interface 500 may include a status bar 510, a tray 520 having a frequently used application icon, and another application icon. The status bar 510 may include a time indicator 5001, a battery status indicator 5002, one or more signal strength indicators 5003 of a wireless fidelity (wireless fidelity, Wi-Fi) signal, and one or more signal strength indicators 5004 of a mobile communication signal (also referred to as a cellular signal). The tray 520 having a frequently used application icon may display: a camera icon 5011, a phone icon 5012, a contacts icon 5013, and a messages icon 5014. The another application icon may be, for example, a clock icon 5005, a calendar icon 5006, a gallery icon 5007, a notes icon 5008, a HUAWEI Video icon 5009, or a health icon 5010. An icon of any application may be used to respond to an operation (for example, a single-tap operation) of the user, so that the terminal device 200 starts the application corresponding to the icon. The health icon 5010 may be used to enable the application Health. The application Health may be used by the terminal device 200 to establish the communication connection relationship with the wearable device 100. The terminal device 200 may display health data of the user, such as a heart rate and a body temperature, to the user by using the application Health.

The terminal device 200 receives and responds to a user operation (for example, a single-tap operation) on the health icon 5010, and the terminal device 200 may display an application interface 600 of Health shown in FIG. 6.

As shown in FIG. 6, the application interface 600 may include the status bar 510 and an interface viewing option 610. The interface viewing option 610 may include a health option 6101, a device option 6102, a discover option 6103, and a me option 6104. Any option may be used to respond to an operation (for example, a single-tap operation) of the user, so that the terminal device 200 displays, on the application interface 600, content corresponding to the option. For example, content corresponding to the device option 6102 may include device information that has been added to the terminal device and a control used to add a new device. When the terminal device 200 detects a user operation (for example, a tap operation) performed on the device option 6102, the terminal device 200 may display an added device option 620 and an add a new device option 630.

The add a new device option 630 may be used to trigger the terminal device 200 to add a new device. The new device is a device that establishes a communication connection relationship with the terminal device 200 for the first time. When the terminal device 200 detects a user operation (for example, a sing-tap operation) performed on the add a new device option 630, the terminal device 200 may display a device adding setting interface, so that the terminal device 200 establishes the communication connection relationship with the new device. The device adding setting interface may be used by the user to search for the new device and a manner of establishing the communication connection, for example, a connection through Bluetooth. A process of establishing the communication connection relationship between the terminal device 200 and the new device is not limited in embodiments of this application.

The added device option 620 may include identifiers of a plurality of wearable devices. The plurality of wearable devices each have established the communication connection relationship with the terminal device 200. For example, the terminal device 200 has established the communication connection relationship with a wearable device A. When detecting a user operation (for example, a single-tap operation) performed on any device option in the added device option 620, the terminal device may display related information corresponding to the device.

When the terminal device 200 detects a user operation (for example, a single-tap operation) performed on the identifier of the wearable device 100 in the added device option 620, the terminal device may display an application interface 700 shown in FIG. 7.

As shown in FIG. 7, the application interface 700 may include the status bar 510, a device status bar 710, and health data 720. The device status bar 710 may be used to display a connection status between the wearable device 100 and the terminal device 200 and a battery level of the wearable device 100. For example, when it is detected that the terminal device 200 establishes the communication connection relationship with the wearable device 100 in a Bluetooth connection manner, the device status bar 710 may prompt that a connection manner is the Bluetooth connection and the connection status is "Connected". Further, the terminal device 200 may obtain battery level information of the wearable device 100. The device status bar 710 may prompt a current battery level (for example, 77%) of the wearable device 100. Content prompted by the device status bar 710 may further include more. This is not limited in embodiments of this application.

The health data 720 may include a quantity of steps that the user moves (for example, 8892 steps), calories burnt (for example, 459 kcal), a distance that the user moves (for example, 5.2 km), a body temperature of the user (for example, 36.7°C), and a heart rate of the user (for example, 94 bpm), and the like that are recorded by the wearable device 100. Content displayed in the health data 720 may further include more. This is not limited in embodiments of this application.

The terminal device 200 responds to a user operation (for example, a single-tap operation) performed on a body temperature option icon 7201, and the terminal device 200 may display a body temperature user interface 800 shown in FIG. 8A to FIG. 8C. Daily body temperature statistics, weekly body temperature statistics, and monthly body temperature statistics may be displayed on the body temperature user interface 800. The body temperature user interface 800 shown in FIG. 8A to FIG. 8C is described by using the daily body temperature statistics as an example. It can be seen from FIG. 8A to FIG. 8C that an average body temperature of the user in a time range from 16:00 p.m. to 17:00 p.m. is 36.7°C. Among the detected body temperatures, a body temperature range of the user is from 36.4 to 37.1°C and a skin temperature range is from 30.9 to 35.3°C. It should be noted that the body temperature of the user shown in FIG. 8A to FIG. 8C is sent to the terminal device 200 after being collected by the wearable device 100.

When the terminal device 200 detects an operation performed on an option 8001 on the user interface 800, the terminal device 200 may display a user interface 801. Two alert modes provided by the terminal device 200 may be displayed on the user interface 801, namely, a high body temperature alert and a low body temperature alert respectively. For example, the high body temperature alert is a default alert manner of a system, and the user may change a default alert manner of the system based on an actual requirement of the user.

When the terminal device 200 detects an operation performed on an option 8011 on the user interface 801, the terminal device 200 may display a user interface 802. Four body temperature monitoring modes provided by the terminal device 200 may be displayed on the user interface 802: which are respectively an intelligent mode, a daily mode, a sleep mode, and an exercise mode. The intelligent mode is a default mode of the system, and the user may determine, based on an actual requirement of the user, a body temperature monitoring mode that the user is in.

In a possible implementation, if the user selects the intelligent mode in the body temperature monitoring mode, the user may customize and adjust, by using an intelligent mode option 8021, a setting related to a fever alarm in the intelligent mode. If the user selects the daily mode in the body temperature monitoring mode, the user may customize and adjust, by using a daily mode option 8022, a setting related to a fever alarm in the daily mode. If the user selects the sleep mode in the body temperature monitoring mode, the user may customize and adjust, by using a sleep mode option 8023, a setting related to a fever alarm in the sleep mode. If the user selects the exercise mode in the body temperature monitoring mode, the user may customize and adjust, by using an exercise mode option 8024, a setting related to a fever alarm in the exercise mode.

After the user determines the body temperature monitoring mode by using the terminal device 200, the terminal device 200 may send, to the wearable device, the instruction for enabling the body temperature monitoring mode, and the wearable device 100 may start the body temperature monitoring mode after specific time (for example, countdown of 3 seconds) after vibration. After the body temperature monitoring mode is enabled, the wearable device may display a body temperature icon and text "Body temperature monitoring mode" on a display 12. In this way, the wearable device 100 may perform a high body temperature alarm based on a body temperature monitoring mode determined by the user.

When receiving the instruction that is sent by the terminal device 200 and that is for disabling the golf mode, the wearable device 100 may disable the body temperature monitoring mode after specific time (for example, countdown of 3 seconds) after vibration.

The following describes four body temperature monitoring modes provided in embodiments of this application.

As shown in FIG. 9, the body temperature monitoring mode includes one or more of the following: an intelligent mode, a daily mode, a sleep mode, and an exercise mode. After a user wears a wearable device 100 for a time period (for example, 10 minutes), the wearable device 100 may continuously collect body temperatures of the user in the time period. When the user determines a body temperature monitoring mode that the user is in, the wearable device 100 may monitor the collected body temperatures of the user in the time period. When the body temperature meets fever time corresponding to the body temperature monitoring mode, the wearable device 100 may display fever alarm information. Details are as follows:

The intelligent mode is a default mode of the wearable device 100, and does not need to be manually adjusted by the user. When the user does not adjust the mode, the wearable device 100 may monitor the body temperature of the user based on the intelligent mode. For example, in the intelligent mode, the wearable device 100 may identify a sleep state or an exercise state of the user, and adjust, in a corresponding state, a fever body temperature and duration of the fever, to determine whether the user has a fever. When determining that the user has a fever risk, the wearable device 100 may display the fever alarm information.

The user can manually select or adjust the daily mode. The mode may cover a low-activity scenario like a home or an office or a resting scenario. After the user manually selects the daily mode, the wearable device 100 may monitor a fever status of the user based on the daily mode. For example, in the daily mode, a single-point truncated solution is still used. In other words, when detecting that the body temperature of the user meets the fever body temperature, the wearable device determines that the user may have the fever risk, and therefore displays the fever alarm information.

The user can manually select or adjust the sleep mode. The mode may cover a sleep scenario. After the user manually selects the sleep mode, the wearable device 100 may monitor the fever status of the user based on the sleep mode. For example, in the sleep mode, the wearable device 100 may determine, based on a setting of the user, an abnormal body temperature and duration that correspond to the sleep mode, and then adjust the abnormal body temperature and the duration based on a detected ambient temperature. When the wearable device 100 detects that the body temperature of the user meets an adjusted abnormal body temperature or adjusted duration, the wearable device 100 determines that the user may have the fever risk, and therefore displays the fever alarm information.

The user can manually select or adjust the exercise mode. The mode may cover an exercise scenario. After the user manually selects the sleep mode, the wearable device 100 may monitor the fever status of the user based on the exercise mode. For example, in the exercise mode, the wearable device 100 may determine, based on a setting of the user, an abnormal body temperature and duration that correspond to the exercise mode, and then adjust the abnormal body temperature and the duration based on a detected exercise state of the user. When the wearable device 100 detects that the body temperature of the user meets an adjusted abnormal body temperature or adjusted duration, the wearable device 100 determines that the user may have the fever risk, and therefore displays the fever alarm information.

### Intelligent mode

FIG. 10A is a diagram of a user interface of an intelligent mode according to an embodiment of this application. After the wearable device 100 shown in FIG. 4B-1 to FIG. 4B-4 receives an operation on the intelligent mode option 4031, or after the terminal device 200 shown in FIG. 8A to FIG. 8C receives an operation on the intelligent mode option 8021, a user interface 1000 of the intelligent mode shown in FIG. 10A may be displayed. It may be understood that, if the wearable device 100 receives and responds to the input of the user to enable the body temperature monitoring mode, the user interface 1000 of the intelligent mode is displayed on the display of the wearable device 100. After the wearable device 100 establishes the communication connection to the terminal device 200, if the wearable device 100 enables the body temperature monitoring mode by receiving the request for enabling the body temperature monitoring mode sent by the terminal device 200, the user interface 1000 of the intelligent mode is displayed on a display of the terminal device 200.

It can be seen from FIG. 10A that the intelligent mode is a default abnormal body temperature alarm mode. In a default mode of the intelligent mode, the wearable device 100 may continuously monitor the body temperature of the user, and when the body temperature is in a fever range for a time period, the wearable device 100 may generate the fever alarm. For example, the wearable device 100 may divide the collected body temperatures of the user into three segments based on 37.2°C, 38°C, and 39°C, where fever time corresponding to 37.2°C is 20 minutes (Minutes, mins), fever time corresponding to 38°C is 5 minutes, and fever time corresponding to 39°C is 1 minute. When detecting that the body temperature of the user meets the corresponding fever time, the wearable device 100 may display the fever alarm information.

In a possible implementation, the user may further customize a fever condition based on an actual requirement of the user. In other words, the user may customize one or more fever ranges and duration corresponding to the fever ranges. In the customized mode of the default mode, the wearable device 100 continuously monitors the body temperature of the user. When the body temperature is in the duration corresponding to the customized fever range, the wearable device 100 may display the fever alarm information.

In a possible implementation, in the intelligent mode, the wearable device may identify the sleep state or the exercise state of the user, and adjust, a fever threshold and duration of the fever threshold that correspond to a corresponding state. If the user does not need to perform body temperature monitoring during exercise or sleep, the user may choose not to prompt during exercise or not to prompt during sleep.

The following uses the default mode of the intelligent mode as an example for description. FIG. 10B-1 and FIG. 10B-2 are a schematic flowchart of the fever alarm in the intelligent mode according to this embodiment of this application. It can be seen from FIG. 10B-1 and FIG. 10B-2 that in a process in which the user continuously wears the wearable device 100, the wearable device 100 may continuously collect the body temperatures of the user.

If detecting that time for which the body temperature of the user exceeds 37.2°C is less than 1 minute, it indicates that the body temperature of the user is in a normal state, and the wearable device 100 does not generate the fever alarm.

If detecting that time for which the body temperature of the user exceeds 37.2°C is greater than 1 minute, and time for which the body temperature of the user exceeds 39°C is greater than 1 minute, it indicates that the user is currently in a high-fever state, and a situation is serious, and the wearable device 100 generates the heat alarm. For example, the wearable device 100 may prompt the user, in a voice or text prompt manner, that the user is currently in the fever state.

If detecting that time for which the body temperature of the user exceeds 37.2°C is greater than 1 minute, and time for which the body temperature of the user exceeds 38°C is greater than 5 minutes, it indicates that the user is currently in a low-fever state, and the wearable device 100 generates the fever alarm. For example, the wearable device 100 may prompt the user, in a voice or text prompt manner, that the user is currently in the fever state.

If detecting that time for which the body temperature of the user exceeds 37.2°C is greater than 1 minute, and time for which the body temperature of the user exceeds 38°C is less than 5 minutes, the wearable device 100 may determine whether the user is in the sleep state. If determining that the user is in the resting, an in-bed, or the sleep state, the wearable device 100 may determine whether the time for which the body temperature of the user exceeds 37.2°C is greater than 10 minutes; and if the time is greater than 10 minutes, it indicates that the user may be in a fever state, and the wearable device 100 generates the fever alarm. For example, the wearable device 100 may prompt the user, in a voice or text prompt manner, that the user is currently in the fever state. If the time for which the body temperature of the user exceeds 37.2°C is less than 10 minutes, it indicates that the user is not in a fever state, and the wearable device 100 does not generate the fever alarm.

If detecting that time for which the body temperature of the user exceeds 37.2°C is greater than 1 minute, and time for which the body temperature of the user exceeds 38°C is less than 5 minutes, the wearable device 100 may determine whether the user is in the sleep state. If the user is not in the sleep state, the wearable device 100 may determine whether the user is in the exercise state. If determining that the user is in the exercise state, the wearable device 100 needs to lower an alarm limit. In other words, if detecting that the time for which the body temperature of the user exceeds 37.2°C is greater than 30 minutes, it indicates that the user may be in a fever state, and the wearable device 100 generates the fever alarm. For example, the wearable device 100 may prompt the user, in a voice or text prompt manner, that the user is currently in the fever state. If the time for which the body temperature of the user exceeds 37.2°C is less than 30 minutes, it indicates that the user is not in a fever state, and the wearable device 100 does not generate the fever alarm.

In the intelligent mode, the user may change, based on living and working environments and an individual difference of the user by using the customized mode, the fever range and the duration corresponding to the fever range. In addition, the user may choose whether to prompt the abnormal body temperature in the sleep state or the exercise state.

FIG. 10C is a diagram of a user interface for generating the fever alarm by the wearable device in the intelligent mode according to this embodiment of this application. It can be seen from FIG. 10C that when the wearable device 100 detects the body temperature of the user is abnormal, for example, the body temperature of the user is higher than 37.5°C in last 20 minutes, the wearable device 100 may prompt the user that there is a risk of abnormal body temperature, and further check and early intervention are required.

### Sleep mode

FIG. 11A is a diagram of a user interface of a sleep mode according to an embodiment of this application. After the wearable device 100 shown in FIG. 4B-1 to FIG. 4B-4 receives an operation on the sleep mode option 4033, or after the terminal device 200 shown in FIG. 8A to FIG. 8C receives an operation on the sleep mode option 8023, a user interface 1100 of the sleep mode shown in FIG. 11A may be displayed. It may be understood that, if the wearable device 100 receives and responds to the input of the user to enable the body temperature monitoring mode, the user interface 1100 of the sleep mode is displayed on the display of the wearable device 100. After the wearable device 100 establishes the communication connection to the terminal device 200, if the wearable device 100 enables the body temperature monitoring mode by receiving the request for enabling the body temperature monitoring mode sent by the terminal device 200, the user interface 1100 of the sleep mode is displayed on a display of the terminal device 200.

It can be seen from FIG. 11A that, in the sleep mode, the user may select a thickness (for example, thin, medium, or thick) of bedding during sleep and whether there is a cold source (for example, whether an air conditioner or an electric fan is turned on). The wearable device 100 may adaptively adjust, based on feedback of the user on the thickness of the bedding and the cold source, an abnormal body temperature threshold and duration corresponding to the abnormal body temperature threshold, to reduce impact on the wearable device 100 caused by covering of the bedding during sleep, and impact of the cold source on the body temperature detection.

In a possible implementation, after the user feeds back the thickness of the bedding and whether the air conditioner or the electric fan is turned on, the wearable device 100 may determine, based on the feedback of the user, a first abnormal body temperature and first duration that correspond to the sleep mode.

In another possible implementation, after the user feeds back the thickness of the bedding and whether the air conditioner or the electric fan is turned on, the user may customize, based on a requirement, a first abnormal body temperature and first duration that correspond to the sleep mode.

An example in which the user customizes the first abnormal body temperature and the first duration is used for description. FIG. 11B is a schematic flowchart of the fever alarm in the sleep mode according to this embodiment of this application. It can be seen from FIG. 11B that in a process in which the user continuously wears the wearable device 100, the wearable device 100 may continuously collect the body temperatures of the user. After the user selects the sleep mode, the user may customize, based on an actual requirement of the user, one or more first abnormal body temperatures and first duration corresponding to the first abnormal body temperature. For example, the first abnormal body temperature and the first duration corresponding to the first abnormal body temperature may include: duration from 36.8°C to 37.2°C is 10 minutes, duration from 37.2°C to 37.5°C is 5 minutes, and duration greater than 37.5°C is 2 minutes.

Subsequently, the user may feed back, based on an actual case of the user, the thickness of the bedding and whether the cold source is turned on. If the feedback of the user is that the thickness of the bedding is small and the cold source is not turned on, when the wearable device 100 monitors whether the user has a fever, the first abnormal body temperature and first abnormal time customized by the user do not need to be changed. In other words, when the wearable device 100 detects that a body temperature in time in which the user wears the wearable device meets the first abnormal time corresponding to the first abnormal body temperature, it indicates that the user may have a fever, and the wearable device 100 may generate the fever alarm.

The feedback of the user is not that the thickness of the bedding is small and the cold source is not turned on, for example, the feedback of the user may include: The thickness of the bedding is medium or large, and the cold source is turned on, the thickness of the bedding is small, and the cold source is turned on, or the thickness of the bedding is medium or large, and the cold source is not turned on. In this case, when monitoring whether the user has a fever, the wearable device 100 needs to dynamically adjust, with reference to an ambient temperature, the first abnormal body temperature and the first duration that are customized by the user. For example, if the feedback of the user is that the thickness of the bedding is not small, and the cold source is turned on, if the wearable device 100 detects that an ambient temperature of the user decreases, it indicates that the wearable device 100 is not covered by the bedding. In this case, the body temperature of the user is normal, but a skin temperature may be low, and it is likely to generate a false fever alarm. Therefore, the wearable device 100 may dynamically decrease the first abnormal body temperature or the first duration customized by the user. In this way, when the wearable device 100 detects, in a case in which the ambient temperature of the user decreases, that a body temperature of the user in the time period in which the user wears the wearable device 100 meets a decreased first abnormal body temperature or prolonged first duration, it indicates that the user may have a fever, and the wearable device 100 may generate the fever alarm.

For example, if the feedback of the user is that the thickness of the bedding is not small, and the cold source is turned on, if the wearable device 100 detects that the ambient temperature of the user increases, it indicates that the wearable device 100 is covered by the bedding. In this case, the body temperature of the user is normal, but the skin temperature may be high, and it is likely to generate the false fever alarm. Therefore, the wearable device 100 may dynamically increase the first abnormal body temperature or shorten the first duration customized by the user. In this way, when the ambient temperature of the user increases, and the wearable device 100 detects that the body temperature of the user in the time period in which the user wears the wearable device 100 meets an increased first abnormal body temperature or shortened first duration, it indicates that the user may have the fever during sleep, and the wearable device 100 may generate the fever alarm.

FIG. 11C is a diagram of a user interface for generating the fever alarm by the wearable device in the sleep mode according to this embodiment of this application. It can be seen from FIG. 11C that when the wearable device 100 detects that the body temperature of the user is abnormal, for example, the body temperature of the user is higher than 37.5°C in last 20 minutes, the wearable device 100 may prompt the user that the body temperature is excessively high during sleep.

### Exercise mode

FIG. 12A is a diagram of a user interface of an exercise mode according to an embodiment of this application. After the wearable device 100 shown in FIG. 4B-1 to FIG. 4B-4 receives an operation on the exercise mode option 4034, or after the terminal device 200 shown in FIG. 8A to FIG. 8C receives an operation on the exercise mode option 8024, a user interface 1200 of the exercise mode shown in FIG. 12A may be displayed. It may be understood that, if the wearable device 100 receives and responds to the input of the user to enable the body temperature monitoring mode, the user interface 1200 of the exercise mode is displayed on the display of the wearable device 100. After the wearable device 100 establishes the communication connection to the terminal device 200, if the wearable device 100 enables the body temperature monitoring mode by receiving the request for enabling the body temperature monitoring mode sent by the terminal device 200, the user interface 1200 of the exercise mode is displayed on a display of the terminal device 200.

It can be seen from FIG. 12A that, in the exercise mode, the wearable device 100 may extract a current exercise state and exercise time of the user, or the user may feed back a current exercise state and exercise time of the user. The wearable device 100 may adaptively adjust, with reference to the extracted exercise state and exercise time, an abnormal body temperature threshold and duration corresponding to the abnormal body temperature threshold, to reduce impact of a false alarm caused by exercise.

In a possible implementation, the wearable device 100 may extract the current exercise state and the exercise time of the user, or the user may input the exercise state and the exercise time of the user. The user may further customize, based on an actual requirement of the user, a third abnormal body temperature and third duration that correspond to the exercise mode.

In another possible implementation, after extracting the exercise state and the exercise time of the user, the wearable device 100 may match a third abnormal body temperature and third duration for the exercise mode.

An example in which the user customizes the third abnormal body temperature and the third duration is used for description. FIG. 12B-1 and FIG. 12B-2 are a schematic flowchart of the fever alarm in the exercise mode according to this embodiment of this application. It can be seen from FIG. 12B-1 and FIG. 12B-2 that in a process in which the user continuously wears the wearable device 100, the wearable device 100 may continuously collect the body temperatures of the user. After the user selects the exercise mode, the user may customize, based on an actual requirement of the user, one or more third abnormal body temperatures and third duration corresponding to the third abnormal body temperature. For example, the third abnormal body temperature and the third duration corresponding to the third abnormal body temperature may include: duration from 37.2°C to 37.8°C is 10 minutes, duration from 37.8°C to 38.5°C is 5 minutes, and duration greater than 38.5°C is 2 minutes.

When the exercise state of the user obtained by the wearable device 100 is a low intensity, the wearable device 100 determines whether the third abnormal body temperature is less than 37.5°C, and when determining that the third abnormal body temperature is less than 37.5°C, the wearable device 100 adaptively adjusts the third abnormal body temperature customized by the user, to increase the third abnormal body temperature, for example, increase the third abnormal body temperature by 0.2°C. When the wearable device 100 determines that the third abnormal body temperature is not less than 37.5°C, the wearable device 100 may set the third abnormal body temperature to 37.5°C. Further, when the exercise state of the user is the low intensity, the wearable device 100 may adaptively adjust the third duration based on exercise time of the user, and may prolong the third duration, for example, prolong the third duration by 10 minutes. Prolonged time needs to be less than or equal to the exercise time of the user.

In this way, when the exercise state of the user is the low intensity, and the wearable device 100 detects that a body temperature of the user in the time period in which the user wears the wearable device 100 meets an adaptively adjusted third abnormal body temperature or third duration, it indicates that the user may have a fever during exercise, and the wearable device 100 may generate the fever alarm.

When the exercise state of the user obtained by the wearable device 100 is a moderate intensity, the wearable device 100 determines whether the third abnormal body temperature is less than 38°C, and when determining that the third abnormal body temperature is less than 38°C, the wearable device 100 adaptively adjusts the third abnormal body temperature customized by the user, to increase the third abnormal body temperature, for example, increase the third abnormal body temperature by 0.2°C. When the wearable device 100 determines that the third abnormal body temperature is not less than 38°C, the wearable device 100 may set the third abnormal body temperature to 38°C. Further, when the exercise state of the user is the moderate intensity, the wearable device 100 may adaptively adjust the third duration based on exercise time of the user, and may prolong the third duration, for example, prolong the third duration by 5 minutes. Prolonged time needs to be less than or equal to the exercise time of the user.

In this way, when the exercise state of the user is the moderate intensity, and the wearable device 100 detects that a body temperature of the user in the time period in which the user wears the wearable device 100 meets an adaptively adjusted third abnormal body temperature or third duration, it indicates that the user may have a fever during exercise, and the wearable device 100 may generate the fever alarm.

When the exercise state of the user obtained by the wearable device 100 is a high intensity, the wearable device 100 determines whether the third abnormal body temperature is less than 38.5°C, and when determining that the third abnormal body temperature is less than 38.5°C, the wearable device 100 adaptively adjusts the third abnormal body temperature customized by the user, to increase the third abnormal body temperature, for example, increase the third abnormal body temperature by 0.2°C. When the wearable device 100 determines that the third abnormal body temperature is not less than 378.5°C, the wearable device 100 may set the third abnormal body temperature to 38.5°C. Further, when the exercise state of the user is the high intensity, the wearable device 100 may adaptively adjust the third duration based on exercise time of the user, and may prolong the third duration, for example, prolong the third duration by 2 minutes. Prolonged time needs to be less than or equal to the exercise time of the user.

In this way, when the exercise state of the user is the high intensity, and the wearable device 100 detects that a body temperature of the user in the time period in which the user wears the wearable device 100 meets an adaptively adjusted third abnormal body temperature or third duration, it indicates that the user may have a fever during exercise, and the wearable device 100 may generate the fever alarm.

It may be understood that the body temperature of the user increases sharply during exercise. It is proposed in a research document that the body temperature during exercise may reach a maximum of 40°C. Therefore, the abnormal body temperature and duration of the abnormal body temperature need to be adaptively adjusted with reference to the exercise state and the exercise time of the user, to avoid generation of a false fever alarm. FIG. 11C is a diagram of a user interface for generating the fever alarm by the wearable device in the exercise mode according to this embodiment of this application. It can be seen from FIG. 12C that when the wearable device 100 detects that the body temperature of the user is abnormal, for example, the body temperature of the user is higher than 38.5°C in last 10 minutes, the wearable device 100 may prompt the user that the user is currently doing high-intensity exercise and the body temperature is excessively high, and drink water properly, to avoid heat stroke.

FIG. 13 is a schematic flowchart of a fever alarm method based on body temperature monitoring according to an embodiment of this application. The method may be applied to a wearable device, and the method includes but is not limited to the following steps.

Step S1301: Collect a body temperature of a user in a first time period.

Specifically, the wearable device provided in this embodiment of this application is a portable device that may be attached to skin of the user. When the wearable device is a smart watch or a smart band, the wearable device may be attached to a wrist part of the user. This facilitates collection of the body temperature of the user. When the wearable device is a smart body temperature sticker, the wearable device may be attached to a skin surface like a forehead or an armpit of the user. This facilitates collection of the body temperature of the user. Therefore, the first time period is time for which the wearable device is attached to the skin of the user.

Step S1302: Determine a body temperature monitoring mode that the user is in.

Specifically, because the body temperature of the human body covers 36°C to 42°C, when the body temperature exceeds 37.2°C, it indicates that there is a fever risk, but the fever may not occur in the human body. Therefore, in this embodiment of this application, four body temperature monitoring modes are provided: which are respectively an intelligent mode, a daily mode, a sleep mode, and an exercise mode, covering scenarios such as daily home, office, sleep, and exercise. Different body temperature monitoring modes correspond to different fever alarm mechanisms. The user may select a corresponding body temperature monitoring mode based on an actual requirement of the user. After the wearable device receives response of the user, the wearable device may determine the body temperature monitoring mode that the user is in.

Step S1303: Generate a fever alarm when detecting that the body temperature of the user in the first time period meets a fever condition corresponding to the body temperature monitoring mode.

Specifically, the fever condition includes one or more body temperatures and time corresponding to each body temperature.

In a possible implementation, if the body temperature monitoring mode selected by the user is the intelligent mode, the wearable device may generate the fever alarm when detecting that the body temperature of the user in the first time period meets a fever condition corresponding to the intelligent mode. It should be noted that the intelligent mode may be the body temperature monitoring mode of the wearable device mode, and the mode is mainly used when the user does not perform modification.

For example, in the intelligent mode, the wearable device may divide an entire abnormal body temperature (body temperatures above 37.2°C) range into three segments based on a first body temperature (for example, 37.2°C), a second body temperature (for example, 38°C), and a third body temperature (for example, 39°C). The first body temperature is less than the second body temperature and less than the third body temperature.

For example, when the wearable device detects that time for which the body temperature of the user in the first time period exceeds the first body temperature (for example, 37.2°C) is less than first time (for example, 1 minute), the wearable device does not generate the fever alarm.

For example, when the wearable device detects that the time for which the body temperature of the user in the first time period exceeds the first body temperature (for example, 37.2°C) is greater than the first time (for example, 1 minute), and detects that time for which the body temperature of the user in the first time period exceeds the third body temperature (for example, 39°C) is greater than third time (for example, 1 minute), the wearable device may generate the fever alarm. The third time is greater than or equal to the first time.

For example, when the wearable device detects that duration in which the body temperature of the user in the first time period exceeds the first body temperature (for example, 37.2°C) is greater than the first time (for example, 1 minute), and detects that time for which the body temperature of the user in the first time period exceeds the second body temperature (for example, 38°C) is greater than second time (for example, 5 minutes), the wearable device may generate the fever alarm. The second time is greater than the first time.

For example, when the wearable device detects that the time for which the body temperature of the user in the first time period exceeds the first body temperature (for example, 37.2°C) is greater than the first time (for example, 1 minute), and detects that the time for which the body temperature of the user in the first time period exceeds the second body temperature (for example, 38°C) is greater than the second time (for example, 5 minutes), the wearable device may determine whether the user is currently in a sleep state. If the wearable device determines that a current state of the user is the sleep state, in the sleep state, the wearable device may determine whether the time for which the body temperature of the user in the first time period is greater than the first body temperature (for example, 37.2°C) exceeds fourth time (for example, 10 minutes). When the wearable device detects that the time for which the body temperature of the user in the first time period exceeds the first body temperature (for example, 37.2°C) is greater than the fourth time (for example, 10 minutes), the wearable device may generate the fever alarm. The fourth time is greater than the first time.

For example, when the wearable device detects that the time for which the body temperature of the user in the first time period exceeds the first body temperature (for example, 37.2°C) is greater than the first time (for example, 1 minute), and detects that the time for which the body temperature of the user in the first time period exceeds the second body temperature (for example, 38°C) is greater than the second time (for example, 5 minutes), the wearable device may determine whether the user is currently in the sleep state. If the wearable device determines that a current state of the user is not the sleep state, the wearable device determines whether the user is currently in an exercise state. If the wearable device determines that a current state of the user is the exercise state, in the exercise state, the wearable device may determine whether the time for which the body temperature of the user in the first time period is greater than the first body temperature (for example, 37.2°C) exceeds fifth time (for example, 30 minutes). When the wearable device detects that the time for which the body temperature of the user in the first time period exceeds the first body temperature (for example, 37.2°C) is greater than the fifth time (for example, 30 minutes), the wearable device may generate the fever alarm. The fifth time is greater than the first time.

For example, when the wearable device detects that the time for which the body temperature of the user in the first time period exceeds the first body temperature (for example, 37.2°C) is greater than the first time (for example, 1 minute), and detects that the time for which the body temperature of the user in the first time period exceeds the second body temperature (for example, 38°C) is greater than the second time (for example, 5 minutes), the wearable device may determine whether the user is currently in the sleep state or the exercise state. If the wearable device determines that a current state of the user is neither the sleep state nor the exercise state, it indicates that the current state of the user may be a daily state. In the daily state, the wearable device may determine whether the time for which the body temperature of the user in the first time period is greater than the first body temperature (for example, 37.2°C) exceeds sixth time (for example, 20 minutes). When the wearable device detects that the time for which the body temperature of the user in the first time period exceeds the first body temperature (for example, 37.2°C) is greater than the sixth time (for example, 2 minutes), the wearable device may generate the fever alarm. The sixth time is greater than the first time.

In another possible implementation, if the body temperature monitoring mode selected by the user is the sleep mode, the wearable device may generate the fever alarm when detecting that the body temperature of the user in the first time period meets a fever condition corresponding to the sleep mode.

For example, in the sleep mode, the user may set a current thickness of bedding and whether there is a cold source, so that impact of the bedding covering the wearable device and the cold source on the body temperature detection in the sleep mode can be reduced. Therefore, the wearable device may determine, based on a setting of the user (for example, a setting about the thickness of the bedding and a setting about whether there is the cold source), one or more first abnormal body temperatures corresponding to the sleep mode and first duration corresponding to the first abnormal body temperature. Optionally, the user may customize and set one or more first abnormal body temperatures and first duration corresponding to the first abnormal body temperature. Therefore, the wearable device may determine, based on the customized setting of the user, the one or more first abnormal body temperatures corresponding to the sleep mode and the first duration corresponding to the first abnormal body temperature. It may be understood that, with reference to the intelligent mode, the one or more first abnormal body temperatures may divide the abnormal body temperatures (for example, the body temperatures exceeding 37.2°C) into a plurality of ranges, and each range has corresponding duration.

The wearable device may adjust the first abnormal body temperature or the first duration based on a detected ambient temperature, to obtain a second abnormal body temperature or second duration. Further, when the wearable device detects that the ambient temperature decreases in preset time (for example, in a short time period, 5 minutes), the wearable device may decrease the first abnormal body temperature to obtain the second abnormal body temperature, for example, the second abnormal body temperature=the first abnormal body temperature-0.2°C, or prolong the first duration to obtain the second duration, for example, the second duration=the first duration+2 minutes. Further, when the wearable device detects that the ambient temperature increases in the preset time (for example, in the short time period, 5 minutes), the wearable device may increase the first abnormal body temperature to obtain the second abnormal body temperature, for example, the second abnormal body temperature=the first abnormal body temperature+0.2°C, or shorten the first duration to obtain the second duration, for example, the second duration=the first duration-2 minutes.

Therefore, the wearable device may generate the fever alarm when detecting that time for which the body temperature of the user in the first time period exceeds the first abnormal body temperature is greater than the second duration;
the wearable device may generate the fever alarm when detecting that time for which the body temperature of the user in the first time period exceeds the second abnormal body temperature is greater than the first duration; or
the wearable device may generate the fever alarm when detecting that the time for which the body temperature of the user in the first time period exceeds the second abnormal body temperature is greater than the second duration.

In another possible implementation, if the body temperature monitoring mode selected by the user is the exercise mode, the wearable device may generate the fever alarm when detecting that the body temperature of the user in the first time period meets a fever condition corresponding to the exercise mode.

For example, in the exercise mode, the user may customize and set one or more third abnormal body temperatures and third duration corresponding to the third abnormal body temperature. Therefore, the wearable device may determine, based on the customized setting of the user, the one or more third abnormal body temperatures corresponding to the exercise mode and the third duration corresponding to the third abnormal body temperature. It may be understood that, with reference to the intelligent mode, the one or more third abnormal body temperatures may divide the abnormal body temperatures (for example, the body temperatures exceeding 37.2°C) into a plurality of ranges, and each range has corresponding duration.

Because there may be a plurality of exercise intensities in an exercise process of the user, and different exercise intensities cause the body temperature of the user to increase to different degrees. Therefore, the wearable device may adjust the third abnormal body temperature or the third duration based on a detected exercise intensity of the user, to obtain a fourth abnormal body temperature or fourth abnormal time. The third abnormal body temperature is less than or equal to the fourth abnormal body temperature, and the third duration is less than the fourth duration.

For example, the exercise intensity may include one or more of low, moderate, and high. If the wearable device detects that the exercise intensity of the user is low, the wearable device may determine whether the body temperature of the user in exercise time is less than 37.5°C; and if the body temperature is less than 37.5°C, increase the third abnormal body temperature to obtain the fourth abnormal body temperature, for example, the fourth abnormal body temperature=the third abnormal body temperature+0.2°C, or if the body temperature is not less than 37.5°C, remain the third abnormal body temperature unchanged, that is, the fourth abnormal body temperature=the third abnormal body temperature. When the exercise state is the low intensity, the wearable device increases the third duration corresponding to the third abnormal body temperature to obtain the fourth duration, for example, the fourth duration=the third duration+10 minutes.

For example, if the wearable device detects that the exercise intensity of the user is moderate, the wearable device may determine whether the body temperature of the user in exercise time is less than 38°C; and if the body temperature is less than 38°C, increase the third abnormal body temperature to obtain the fourth abnormal body temperature, for example, the fourth abnormal body temperature=the third abnormal body temperature+0.2°C, or if the body temperature is not less than 38°C, remain the third abnormal body temperature unchanged, that is, the fourth abnormal body temperature=the third abnormal body temperature. When the exercise state is the moderate intensity, the wearable device increases the third duration corresponding to the third abnormal body temperature to obtain the fourth duration, for example, the fourth duration=the third duration+5 minutes.

For example, if the wearable device detects that the exercise intensity of the user is high, the wearable device may determine whether the body temperature of the user in exercise time is less than 38.5°C; and if the body temperature is less than 38.5°C, increase the third abnormal body temperature to obtain the fourth abnormal body temperature, for example, the fourth abnormal body temperature=the third abnormal body temperature+0.2°C, or if the body temperature is not less than 38°C, remain the third abnormal body temperature unchanged, that is, the fourth abnormal body temperature=the third abnormal body temperature. When the exercise state is the high intensity, the wearable device increases the third duration corresponding to the third abnormal body temperature to obtain the fourth duration, for example, the fourth duration=the third duration+2 minutes.

Therefore, after the wearable device adjusts the third abnormal body temperature and the third duration based on the detected exercise intensity of the user, and obtains the fourth abnormal body temperature and the fourth duration, the wearable device may generate the fever alarm when detecting that time for which the body temperature of the user in the exercise time in the first time period exceeds the third abnormal body temperature is greater than the fourth duration;
after the wearable device adjusts the third abnormal body temperature and the third duration based on the detected exercise intensity of the user, and obtains the fourth abnormal body temperature and the fourth duration, the wearable device may generate the fever alarm when detecting that time for which the body temperature of the user in the exercise time in the first time period exceeds the fourth abnormal body temperature is greater than the third duration; or
after the wearable device adjusts the third abnormal body temperature and the third duration based on the detected exercise intensity of the user, and obtains the fourth abnormal body temperature and the fourth duration, the wearable device may generate the fever alarm when detecting that the time for which the body temperature of the user in the exercise time in the first time period exceeds the fourth abnormal body temperature is greater than the fourth duration.

It may be understood that the wearable device may extract the exercise time of the user, and the body temperature monitoring in the exercise mode is performed based on the body temperature of the user extracted in the exercise time.

In this application, the first body temperature, the second body temperature, the third body temperature, the first abnormal body temperature, the second abnormal body temperature, the third abnormal body temperature, and the fourth abnormal body temperature are all fever body temperatures (for example, the body temperatures greater than 37.2°C) in a conventional sense.

It should be noted that, in embodiments of this application, "at least one" means one or more, and "a plurality of" means two or more. Unless otherwise defined, all technical and scientific terms used in this specification have same meanings as those usually understood by a person skilled in the art of this application. Terms used in the specification of this application are merely intended to describe specific embodiments, but are not intended to limit this application. It should be understood that, in this application, unless otherwise specified, "/" means or. For example, A/B may indicate A or B. In this application, a term "and/or" describes only an association relationship between associated objects and indicates that three relationships may exist. It should be noted that in embodiments of this application, terms such as "first" and "second" are merely used for distinguishing and description, and should not be understood as indicating or implying relative importance, and should not be understood as indicating or implying a sequence. A feature limited by "first" or "second" may explicitly or implicitly include one or more features. In descriptions of embodiments of this application, words such as "example" or "for example" are used to indicate giving an example, an illustration, or a description. Any embodiment or design scheme described as an "example" or "for example" in embodiments of this application should not be explained as being more preferred or having more advantages than another embodiment or design scheme. Exactly, use of the words such as "example" or "for example" is intended to present a relative concept in a specific manner.

A person of ordinary skill in the art may understand that all or some of the processes of the methods in the foregoing embodiments may be implemented by a computer program instructing related hardware. The computer program may be stored in a computer-readable storage medium. When the computer program is run, the processes in the foregoing method embodiments are performed. The storage medium includes any medium that can store computer program code, for example, a read-only memory (read-only memory, ROM), a random access memory (random access memory, RAM), a magnetic disk, or an optical disc.

## Claims

1. A fever alarm method based on body temperature monitoring, applied to a wearable device (100), the wearable device (100) comprising a temperature sensor (130K), wherein the method comprises:
collecting (S1301) a body temperature of a user in a first time period, wherein the first time period is time for which the wearable device (100) is attached to skin of the user;
determining (S1302) a body temperature monitoring mode that the user is in; and
generating (S1303) a fever alarm when detecting that the body temperature of the user in the first time period meets a fever condition corresponding to the body temperature monitoring mode, wherein the fever condition comprises one or more body temperatures and time corresponding to each body temperature, and different body temperature monitoring modes correspond to different fever conditions.
wherein the body temperature monitoring mode comprises an intelligent mode;
wherein if the body temperature monitoring mode that the user is in is the intelligent mode, the generating (S1303) a fever alarm when detecting that the body temperature of the user in the first time period meets a fever condition corresponding to the body temperature monitoring mode comprises:
in a case of detecting that time for which the body temperature of the user in the first time period exceeds a first body temperature is greater than first time, when detecting that time for which the body temperature of the user in the first time period exceeds a third body temperature is greater than third time, generating the fever alarm, wherein the third body temperature is greater than the first body temperature, and the third time is greater than or equal to the first time.
wherein the method further comprises:
in a case of detecting that the time for which the body temperature of the user in the first time period exceeds the first body temperature is greater than the first time, when detecting that time for which the body temperature of the user in the first time period exceeds a second body temperature is greater than second time, generating the fever alarm, wherein the second body temperature is greater than the first body temperature, and the second time is greater than the first time; and
**characterised in that** the method further comprises:
in a case of detecting that the time for which the body temperature of the user in the first time period exceeds the first body temperature is greater than the first time, when detecting that time for which the body temperature of the user in the first time period exceeds a second body temperature is less than second time, determining that a current state of the user is a sleep state; and
in the sleep state, generating the fever alarm when detecting that the time for which the body temperature of the user in the first time period exceeds the first body temperature is greater than fourth time, wherein the fourth time is greater than the first time.

2. The method according to claim 1, further comprising:
in a case of detecting that the time for which the body temperature of the user in the first time period exceeds the first body temperature is greater than the first time, when detecting that the time for which the body temperature of the user in the first time period exceeds the second body temperature is less than the second time, determining that the current state of the user is an exercise state; and
in the exercise state, generating the fever alarm when detecting that the time for which the body temperature of the user in the first time period exceeds the first body temperature is greater than fifth time, wherein the fifth time is greater than the first time.

3. The method according to claim 1 or 2, further comprising:
in a case of detecting that the time for which the body temperature of the user in the first time period exceeds the first body temperature is greater than the first time, when detecting that the time for which the body temperature of the user in the first time period exceeds the second body temperature is less than the second time, determining that the current state of the user is a daily state; and
in the daily state, generating the fever alarm when detecting that the time for which the body temperature of the user in the first time period exceeds the first body temperature is greater than sixth time, wherein the sixth time is greater than the first time.

4. A wearable device (100), comprising a temperature sensor (130K), a transceiver, a processor, and a memory, wherein the memory is configured to store a computer program, and the processor invokes the computer program to cause the wearable device (100) to perform the method according to any one of claims 1 to 3.

5. A computer storage medium, wherein the computer storage medium stores a computer program, and when the computer program is executed by a processor, the computer program causes the wearable device (100) of claim 4 to execute the steps of the method according to any one of claims 1 to 3.

## Patentansprüche

1. Fieberalarmverfahren, das auf Körpertemperaturüberwachung basiert und auf eine am Körper zu tragende Vorrichtung (100) angewendet wird, wobei die am Körper zu tragende Vorrichtung (100) einen Temperatursensor (130K) umfasst, wobei das Verfahren umfasst:
Erfassen (S1301) einer Körpertemperatur eines Benutzers in einer ersten Zeitspanne, wobei die erste Zeitspanne eine Zeit ist, in der die am Körper zu tragende Vorrichtung (100) an der Haut des Benutzers angebracht ist;
Bestimmen (S1302) eines Körpertemperaturüberwachungsmodus, in dem sich der Benutzer befindet; und
Erzeugen (S1303) eines Fieberalarms, wenn erkannt wird, dass die Körpertemperatur des Benutzers in der ersten Zeitspanne eine Fieberbedingung erfüllt, die dem Körpertemperaturüberwachungsmodus entspricht, wobei die Fieberbedingung eine oder mehrere Körpertemperaturen und eine jeder Körpertemperatur entsprechende Zeit umfasst und verschiedene Körpertemperaturüberwachungsmodi verschiedenen Fieberbedingungen entsprechen,
wobei der Körpertemperaturüberwachungsmodus einen intelligenten Modus umfasst; wobei, wenn der Körpertemperaturüberwachungsmodus, in dem sich der Benutzer befindet, der intelligente Modus ist, das Erzeugen (S1303) eines Fieberalarms beim Erkennen, dass die Körpertemperatur des Benutzers in der ersten Zeitspanne eine Fieberbedingung erfüllt, die dem Körpertemperaturüberwachungsmodus entspricht, umfasst:
in einem Fall, dass erkannt wird, dass eine Zeit, für die die Körpertemperatur des Benutzers in der ersten Zeitspanne eine erste Körpertemperatur übersteigt, größer als eine erste Zeit ist, wenn erkannt wird, dass eine Zeit, für die die Körpertemperatur des Benutzers in der ersten Zeitspanne eine dritte Körpertemperatur übersteigt, größer als eine dritte Zeit ist, Erzeugen des Fieberalarms, wobei die dritte Körpertemperatur größer als die erste Körpertemperatur ist und die dritte Zeit größer oder gleich der ersten Zeit ist,
wobei das Verfahren ferner umfasst:
in einem Fall, dass erkannt wird, dass die Zeit, für die die Körpertemperatur des Benutzers in der ersten Zeitspanne die erste Körpertemperatur übersteigt, größer ist als die erste Zeit, wenn erkannt wird, dass eine Zeit, für die die Körpertemperatur des Benutzers in der ersten Zeitspanne eine zweite Körpertemperatur übersteigt, größer ist als eine zweite Zeit, Erzeugen des Fieberalarms, wobei die zweite Körpertemperatur größer ist als die erste Körpertemperatur und die zweite Zeit größer ist als die erste Zeit; und
**dadurch gekennzeichnet, dass** das Verfahren ferner umfasst:
in einem Fall, dass erkannt wird, dass die Zeit, für die die Körpertemperatur des Benutzers in der ersten Zeitspanne die erste Körpertemperatur übersteigt, größer als die erste Zeit ist, wenn erkannt wird, dass eine Zeit, für die die Körpertemperatur des Benutzers in der ersten Zeitspanne eine zweite Körpertemperatur übersteigt, kleiner als eine zweite Zeit ist, Bestimmen, dass ein aktueller Zustand des Benutzers ein Schlafzustand ist; und
im Schlafzustand, Erzeugen des Fieberalarms, wenn erkannt wird, dass die Zeit, für die die Körpertemperatur des Benutzers in der ersten Zeitspanne die erste Körpertemperatur überschreitet, größer ist als eine vierte Zeit, wobei die vierte Zeit größer ist als die erste Zeit.

2. Verfahren nach Anspruch 1, ferner umfassend:
in einem Fall, dass erkannt wird, dass die Zeit, für die die Körpertemperatur des Benutzers in der ersten Zeitspanne die erste Körpertemperatur übersteigt, größer als die erste Zeit ist, wenn erkannt wird, dass die Zeit, für die die Körpertemperatur des Benutzers in der ersten Zeitspanne die zweite Körpertemperatur übersteigt, kleiner als die zweite Zeit ist, Bestimmen, dass der aktuelle Zustand des Benutzers ein Zustand der sportlichen Betätigung ist; und
im Zustand der sportlichen Betätigung, Erzeugen des Fieberalarms, wenn erkannt wird, dass die Zeit, für die die Körpertemperatur des Benutzers in der ersten Zeitspanne die erste Körpertemperatur überschreitet, größer ist als eine fünfte Zeit, wobei die fünfte Zeit größer ist als die erste Zeit.

3. Verfahren nach Anspruch 1 oder 2, ferner umfassend:
in einem Fall, dass erkannt wird, dass die Zeit, für die die Körpertemperatur des Benutzers in der ersten Zeitspanne die erste Körpertemperatur übersteigt, größer als die erste Zeit ist, wenn erkannt wird, dass die Zeit, für die die Körpertemperatur des Benutzers in der ersten Zeitspanne die zweite Körpertemperatur übersteigt, kleiner als die zweite Zeit ist, Bestimmen, dass der aktuelle Zustand des Benutzers ein Tageszustand ist; und
im Tageszustand, Erzeugen des Fieberalarms, wenn erkannt wird, dass die Zeit, für die die Körpertemperatur des Benutzers in der ersten Zeitspanne die erste Körpertemperatur überschreitet, größer ist als eine sechste Zeit, wobei die sechste Zeit größer ist als die erste Zeit.

4. Am Körper zu tragende Vorrichtung (100), umfassend einen Temperatursensor (130K), einen Sender-Empfänger, einen Prozessor und einen Speicher, wobei der Speicher so ausgelegt ist, dass er ein Computerprogramm speichert, und der Prozessor das Computerprogramm aufruft, um die am Körper zu tragende Vorrichtung (100) zu veranlassen, das Verfahren nach einem der Ansprüche 1 bis 3 durchzuführen.

5. Computerspeichermedium, wobei das Computerspeichermedium ein Computerprogramm speichert und, wenn das Computerprogramm von einem Prozessor ausgeführt wird, das Computerprogramm die am Körper zu tragende Vorrichtung (100) nach Anspruch 4 veranlasst, die Schritte des Verfahrens nach einem der Ansprüche 1 bis 3 auszuführen.

## Revendications

1. Procédé d'alarme de fièvre basé sur une surveillance de la température corporelle, appliqué à un dispositif à porter sur soi (100), le dispositif à porter sur soi (100) comprenant un capteur de température (130K), le procédé comprenant :
la collecte (S1301) d'une température corporelle d'un utilisateur au cours d'une première période de temps, la première période de temps étant une durée pendant laquelle le dispositif à porter sur soi (100) est attaché à la peau de l'utilisateur ;
la détermination (S1302) d'un mode de surveillance de la température corporelle dans lequel se trouve l'utilisateur ; et
la génération (S1303) d'une alarme de fièvre lors de la détection que la température corporelle de l'utilisateur au cours de la première période de temps remplit une condition de fièvre correspondant au mode de surveillance de la température corporelle, la condition de fièvre comprenant une ou plusieurs températures corporelles et une durée correspondant à chaque température corporelle, et des modes de surveillance de la température corporelle différents correspondant à des conditions de fièvre différentes,
dans lequel le mode de surveillance de la température corporelle comprend un mode intelligent ;
dans lequel, si le mode de surveillance de la température corporelle dans lequel se trouve l'utilisateur est le mode intelligent, la génération (S1303) d'une alarme de fièvre lors de la détection que la température corporelle de l'utilisateur au cours de la première période de temps remplit une condition de fièvre correspondant au mode de surveillance de la température corporelle comprend :
en cas de détection qu'une durée pendant laquelle la température corporelle de l'utilisateur au cours de la première période de temps dépasse une première température corporelle est supérieure à une première durée, lors de la détection qu'une durée pendant laquelle la température corporelle de l'utilisateur au cours de la première période de temps dépasse une troisième température corporelle est supérieure à une troisième durée, la génération de l'alarme de fièvre, la troisième température corporelle étant supérieure à la première température corporelle, et la troisième durée étant supérieure ou égale à la première durée ;
le procédé comprenant en outre :
en cas de détection que la durée pendant laquelle la température corporelle de l'utilisateur au cours de la première période de temps dépasse la première température corporelle est supérieure à la première durée, lors de la détection qu'une durée pendant laquelle la température corporelle de l'utilisateur au cours de la première période de temps dépasse une deuxième température corporelle est supérieure à une deuxième durée, la génération de l'alarme de fièvre, la deuxième température corporelle étant supérieure à la première température corporelle, et la deuxième durée étant supérieure à la première durée ; et
le procédé étant **caractérisé en ce qu'**il comprend en outre :
en cas de détection que la durée pendant laquelle la température corporelle de l'utilisateur au cours de la première période de temps dépasse la première température corporelle est supérieure à la première durée, lors de la détection qu'une durée pendant laquelle la température corporelle de l'utilisateur au cours de la première période de temps dépasse une deuxième température corporelle est inférieure à une deuxième durée, la détermination qu'un état courant de l'utilisateur est un état de sommeil ; et
dans l'état de sommeil, la génération de l'alarme de fièvre lors de la détection que la durée pendant laquelle la température corporelle de l'utilisateur au cours de la première période de temps dépasse la première température corporelle est supérieure à une quatrième durée, la quatrième durée étant supérieure à la première durée.

2. Procédé selon la revendication 1, comprenant en outre :
en cas de détection que la durée pendant laquelle la température corporelle de l'utilisateur au cours de la première période de temps dépasse la première température corporelle est supérieure à la première durée, lors de la détection que la durée pendant laquelle la température corporelle de l'utilisateur au cours de la première période de temps dépasse la deuxième température corporelle est inférieure à la deuxième durée, la détermination que l'état courant de l'utilisateur est un état d'exercice ; et
dans l'état d'exercice, la génération de l'alarme de fièvre lors de la détection que la durée pendant laquelle la température corporelle de l'utilisateur au cours de la première période de temps dépasse la première température corporelle est supérieure à une cinquième durée, la cinquième durée étant supérieure à la première durée.

3. Procédé selon la revendication 1 ou 2, comprenant en outre :
en cas de détection que la durée pendant laquelle la température corporelle de l'utilisateur au cours de la première période de temps dépasse la première température corporelle est supérieure à la première durée, lors de la détection que la durée pendant laquelle la température corporelle de l'utilisateur au cours de la première période de temps dépasse la deuxième température corporelle est inférieure à la deuxième durée, la détermination que l'état courant de l'utilisateur est un état quotidien ; et
dans l'état quotidien, la génération de l'alarme de fièvre lors de la détection que la durée pendant laquelle la température corporelle de l'utilisateur au cours de la première période de temps dépasse la première température corporelle est supérieure à une sixième durée, la sixième durée étant supérieure à la première durée.

4. Dispositif à porter sur soi (100), comprenant un capteur de température (130K), un émetteur-récepteur, un processeur, et une mémoire, la mémoire étant configurée pour stocker un programme informatique, et le processeur invoquant le programme informatique afin d'amener le dispositif à porter sur soi (100) à réaliser le procédé selon l'une quelconque des revendications 1 à 3.

5. Support de stockage informatique, le support de stockage informatique stockant un programme informatique et, lorsque le programme informatique est exécuté par un processeur, le programme informatique amenant le dispositif à porter sur soi (100) selon la revendication 4 à exécuter les étapes du procédé selon l'une quelconque des revendications 1 à 3.
